# EUROPEAN PATENT SPECIFICATION

(11) **EP 1 337 558 B1**
(45) Date of publication and mention of the grant of the patent: **01.09.2010**
(21) Application number: 01980733.8
(22) Date of filing: 07.11.2001
(51) Int. Cl.: C07K 14/705, C12N 15/12

(54) **A "BACH" G PROTEIN COUPLED RECEPTOR POLYPEPTIDE AND POLYNUCLEOTIDE ENCODING THIS RECEPTOR**
EIN "BACH" G-PROTEIN GEKOPPELTER REZEPTOR UND DAFÜR KODIERENDE POLYNUCLEOTIDE
UN RECEPTEUR "BACH" COUPLE AUX PROTEINES G ET POLYNUCLEOTIDES CODANT POUR CES POLYPEPTIDES

(30) Priority: 07.11.2000 GB 0027170; 14.11.2000 US 248411 P
(43) Date of publication of application: 27.08.2003
(73) Proprietor: Takeda Pharmaceutical Company Limited, Osaka-shi, Osaka 541-0045 (JP)
(72) Inventor: APARICIO, Samuel, Paradigm Therapeutics Limited, Cambridge CB4 0GP (GB); CARLTON, Mark, Paradigm Therapeutics Limited, Cambridge CB4 0GP (GB); DIXON, John, Paradigm Therapeutics Limited, Cambridge CB4 0GP (GB); MESSAGER, Sophie, Paradigm Therapeutics Limited, Cambridge CB4 0GP (GB); RUSS, Andreas, Paradigm Therapeutics Limited, Cambridge CB4 0GP (GB); THRESHER, Rosemary, Paradigm Therapeutics Limited, Cambridge CB4 0GP (GB)
(74) Representative: Jones, Nicholas Andrew
(86) International application number: PCT/GB2001/004935
(87) International publication number: WO 2002/038607

(56) References cited:
- WO-A-00/22131
- WO-A-00/23588
- WO-A-00/31258
- DATABASE EMBL/GENBANK [Online] Accession number AC AJ272207, ID HSA272207, 16 August 2000 (2000-08-16) STROM T.M.: "G protein-coupled receptor 92, GPCR 92 gene" XP002203948
- MILLIGAN G: "NEW ASPECTS OF G-PROTEIN-COUPLED RECEPTOR SIGNALLING AND REGULATION" TRENDS IN ENDOCRINOLOGY AND METABOLISM, ELSEVIER SCIENCE PUBLISHING, NEW YORK, NY, US, vol. 9, no. 1, January 1998 (1998-01), pages 13-19, XP001056780 ISSN: 1043-2760
- ZINGONI ALESSANDRA ET AL: "Isolation and chromosomal localization of GPR31, a human gene encoding a putative G protein-coupled receptor" GENOMICS, ACADEMIC PRESS, SAN DIEGO, US, vol. 42, no. 3, 1997, pages 519-523, XP002174048 ISSN: 0888-7543
- WILSON S ET AL: "ORPHAN G-PROTEIN-COUPLED RECEPTORS: THE NEXT GENERATION OF DRUG TARGETS?" BRITISH JOURNAL OF PHARMACOLOGY, BASINGSTOKE, HANTS, GB, vol. 125, no. 7, December 1998 (1998-12), pages 1387-1392, XP001010584 ISSN: 0007-1188
- STADEL ET AL: "Orphan G protein-coupled receptors:" TRENDS IN PHARMACOLOGICAL SCIENCES, ELSEVIER TRENDS JOURNAL, CAMBRIDGE, GB, no. 18, 1 November 1997 (1997-11-01), pages 430-437, XP002073279 ISSN: 0165-6147
- RHEE VAN A M ET AL: "MOLECULAR ARCHITECTURE OF G PROTEIN-COUPLED RECEPTORS" DRUG DEVELOPMENT RESEARCH, NEW YORK, NY, US, vol. 37, no. 1, January 1996 (1996-01), pages 1-38, XP001011411 ISSN: 0272-4391

## Description

### FIELD

This invention relates to newly identified nucleic acids, polypeptides encoded by them and to their production and use. More particularly, the nucleic acids and polypeptides of the present invention relate to a G-protein coupled receptor (GPCR), hereinafter referred to as "BACH GPCR", and members of the purinoceptor family of GPCRs. The invention also relates to inhibiting or activating the action of such nucleic acids and polypeptides.

### BACKGROUND

It is well established that many medically significant biological processes are mediated by proteins participating in signal transduction pathways that involve G-proteins and/or second messengers, for example, cAMP (Lefkowitz, Nature, 1991, 351: 353-354). These proteins are referred to as proteins participating in pathways with G-proteins or "PPG proteins". Some examples of these proteins include the GPC receptors, such as those for adrenergic agents and dopamine (Kobilka, B. K., et al., Proc. Natl Acad. Sci., USA, 1987, 84: 46-50; Kobilka B. K., et al., Science, 1987,238: 650-656; Bunzow, J. R., et al., Nature, 1988, 336: 783-787), G-proteins themselves, effector proteins, for example, phospholipase C, adenyl cyclase, and phosphodiesterase, and actuator proteins, for example, protein kinase A and protein kinase C (Simon, M. I., et al., Science, 1991, 252: 802-8).

For example, in one form of signal transduction, the effect of hormone binding is activation of the enzyme adenylate cyclase inside the cell. Enzyme activation by hormones is dependent on the presence of the nucleotide, GTP. GTP also influences hormone binding. A G-protein connects the hormone receptor to adenylate cyclase. G-protein is shown to exchange GTP for bound GDP when activated by a hormone receptor. The GTP carrying form then binds to activated adenylate cyclase. Hydrolysis of GTP to GDP, catalysed by the G-protein itself, returns the G-protein to its basal, inactive form. Thus, the G-protein serves a dual role, as an intermediate that relays the signal from receptor to effector, and as a clock that controls the duration of the signal.

The membrane protein gene superfamily of G-protein coupled receptors (GPCRs) has been characterised as having seven putative transmembrane domains. The domains are believed to represent transmembrane α-helices connected by extracellular or cytoplasmic loops. G-protein coupled receptors include a wide range of biologically active receptors, such as hormone, viral, growth factor and neuroreceptors.

G-protein coupled receptors (also known as 7TM receptors) have been characterised as including these seven conserved hydrophobic stretches of about 20 to 30 amino acids, connecting at least eight divergent hydrophilic loops. The G-protein family of coupled receptors includes dopamine receptors which bind to neuroleptic drugs used for treating psychotic and neurological disorders. Other examples of members of this family include, but are not limited to, calcitonin, adrenergic, endothelin, cAMP, adenosine, muscarinic, acetylcholine, serotonin, histamine, thrombin, kinin, follicle stimulating hormone, opsins, endothelial differentiation gene-1, rhodopsins, odorant, and cytomegalovirus receptors.

Most G-protein coupled receptors have single conserved cysteine residues in each of the first two extracellular loops which form disulphide bonds that are believed to stabilise functional protein structure. The 7 transmembrane regions are designated as TM1, TM2, TM3, TM4, TM5, TM6, and TM7. TM3 has been implicated in signal transduction.

Phosphorylation and lipidation (pamitylation or farnesylation) of cysteine residues can influence signal transduction of some G-protein coupled receptors. Most G-protein coupled receptors contain potential phosphorylation sites within the third cytoplasmic loop and/or the carboxy terminus. For several G-protein coupled receptors, such as the β - adrenoreceptor, phosphorylation by protein kinase A and/or specific receptor kinases mediates receptor desensitization. For some receptors, the ligand binding sites of G-protein coupled receptors are believed to comprise hydrophilic sockets formed by several G-protein coupled receptor transmembrane domains, the sockets being surrounded by hydrophobic residues of the G-protein coupled receptors. The hydrophilic side of each G-protein coupled receptor transmembrane helix is thought to face inward and form a polar ligand binding site. TM3 has been implicated in several G-protein coupled receptors as having a ligand binding site, such as the TM3 aspartate residue. TM5 serines, a TM6 asparagine and TM6 or TM7 phenylalanines or tyrosines are also implicated in ligand binding.

G-protein coupled receptors can be intracellularly coupled by heterotrimeric G-proteins to various intracellular enzymes, ion channels and transporters (see, Johnson et al., Endoc. Rev., 1989, 10: 317-331). Different G-protein α-subunits preferentially stimulate particular effectors to modulate various biological functions in a cell. Phosphorylation of cytoplasmic residues of G-protein coupled receptors has been identified as an important mechanism for the regulation of G-protein coupling of some G-protein coupled receptors. G-protein coupled receptors are found in numerous sites within a mammalian host. Over the past 15 years, nearly 350 therapeutic agents targeting 7 transmembrane (7 TM) receptors have been successfully introduced onto the market.

Thus, G-protein coupled receptors have an established, proven history as therapeutic targets. Clearly there is a need for identification and characterization of further receptors which can play a role in preventing, ameliorating or correcting dysfunctions or diseases, including, but not limited to, infections such as bacterial, fungal, protozoan and viral infections, particularly infections caused by HIV-1 or HIV-2; pain; cancers; diabetes, obesity; anorexia; bulimia; asthma; Parkinson's disease; thrombosis; acute heart failure; hypotension; hypertension; erectile dysfunction; urinary retention; metabolic bone diseases such as osteoporisis and osteo petrosis; angina pectoris; myocardial infarction; ulcers; asthma; allergies; rheumatoid arthritis; inflammatory bowel disease; irritable bowel syndrome benign prostatic hypertrophy; and psychotic and neurological disorders, including anxiety, schizophrenia, manic depression, delirium, dementia, severe mental retardation and dyskinesias, such as Huntington's disease or Gilles dela Tourett's syndrome.

### SUMMARY

According to a first aspect of the present invention, we provide an *in vitro* method of identifying a molecule suitable for the treatment or alleviation of pain or a disorder of urogenital function the method comprising exposing a candidate molecule to a cell expressing a BACH polypeptide comprising an amino acid sequence shown in SEQ ID NO: 3, SEQ ID NO: 5. SEQ In NO: 7 or SEO ID NO: 9 or a functionally equivalent sequence having at least 90% sequence identity thereto and determining if a candidate molecule is an agonist or antagonist of the BACH polypeptide.

There is provided, according to a second aspect of the present invention, a method for providing an indication useful in the diagnosis of or a determination of susceptibility to pain or a disorder of urogenital function in an individual, the method comprising detecting a change in the expression pattern or level of a BACH polypeptide having an amino acid sequence shown in SEQ ID NO: 3. SEQ ID NO: 5, SEQ ID NO: 7 or SEQ ID NO: 9 or a functionally equivalent sequence having at least 90% sequence identity thereto, in a sample from the individual.

We provide, according to a third aspect of the present invention, a method for providing an indication useful in the diagnosis of or a determination of susceptibility to pain or a disorder of urogenital function in an individual, the method comprising detecting a polymorphism in a BACH polynucleotide comprising a nucleic acid sequence shown in SEQ ID NO: 1, SEQ ID NO: 2, SEQ ID NO: 4 or SEQ ID NO: 10 or a functionally equivalent sequence having at least 90% sequence identity thereto, in a sample from the individual.

As a fourth aspect of the present invention, there is provided use of a non-human animal which displays a decrease in sensitivity to pain or a hypoactive bladder when compared to a wild-type animal, the non-human animal being a transgenic animal having a functionally disrupted endogenous BACH gene, wherein the BACH gene comprises a nucleic acid sequence shown in SEQ ID NO: 1, SEQ ID NO: 2. SEQ ID NO: 4, SEQ ID NO: 10 or a functionally equivalent sequence having at least 90% sequence identity thereto, to identify an agonist or antagonist of the BACH polypeptide for the treatment or alleviation of pain or a disorder of urogenital function.

These and other embodiments of the invention will be described in further detail below.

### BRIEF DESCRIPTION OF THE DRAWINGS

Figure 1 is a diagram showing the results of analysis of the human BACH polypeptide (SEQ ID NO: 3) using the HMM structural prediction software of pfam (http://www.sanger.ac.uk/Software/Pfam/search.shtml).
Figure 2 is a diagram showing an expression profile for human BACH GPCR generated by reverse transcription-polymerase chain reaction (RT-PCR).
Figure 3 shows a restriction map of vector pTK3IBLMNL used in the construction of transgenic BACH knock-out mice.
Figure 4 shows the structure of an allele of BACH in which the transmembrane regions are replaced by a lacZ reporter gene and a selectable marker; BACH knock-out mouse may be prepared comprising this allele using the methods described in the Examples.
Figure 5 shows the structure of a wild type allele of BACH.
Figure 6 show sections of dorsal root ganglion taken from BACH knockout mice. Panel A: 100 x magnification; Panel B: 200 x magnification; Panel C 400 x magnification. Closed arrows indicate LazZ stained cells and open arrows indicate cells that do not express LacZ.
Figure 7 shows BACH GPCR expression in sensory neurons. Left hand panels: BACH expression in dorsal root ganglia; Right hand panels: BACH expression in trigeminal ganglia.
Figure 8 (reference example) shows Rotarod active performance of BACH knockout mice. Panel A shows performance of mice with outbred background (three trials), Panel B shows performance of mice with inbred background (four trials). Light grey bars: mice having-/- genotype, dark grey bars: mice having +/-genotype, white bars: mice having +/+genotype.
   Y axis: time in seconds.
Figure 9 shows results of a paw pressure test. X axis: mice having-/-genotype, mice having +/-genotype, mice having +/+ genotype. Y axis: withdrawal response (0 = no withdrawal, 1 = slow withdrawal of the paw, 2 = medium withdrawal of the paw, 3 = fast withdrawal of the paw).

### Sequence Listings

**SEQ ID NO: 1** shows the cDNA sequence of human BACH. **SEQ ID NO: 2** shows an open reading frame derived from SEQ ID NO: 1. **SEQ ID NO: 3** shows the amino acid sequence of human BACH. **SEQ ID NO: 4** shows the open reading frame of a cDNA for Mouse BACH. **SEQ ID NO: 5** shows the amino acid sequence of Mouse BACH. **SEQ ID NO: 6** shows the nucleic acid sequence of a BACH fragment which is cloned into a pTOPO-Echo Donor vector to produce an expression construct. The expression construct produces a polypeptide having an amino acid sequence shown in **SEQ ID NO: 7. SEQ ID NO: 8** shows the nucleic acid sequence of a BACH fragment which is cloned into a pcDNA 3.1 A Myc/His vector to produce an expression construct. The expression construct produces a polypeptide having an amino acid sequence shown in **SEQ ID NO: 9.**

A genomic sequence of mouse BACH is shown in the section headed "BACH Genomic Sequence" (SEQ ID NO: 10). Such a sequence may be used to prepare BACH knock out mice as described below and in the Examples.

### DETAILED DESCRIPTION

### BACH GPCR

Our invention relates in general to a novel G-Protein Coupled Receptor (GPCR), in particular, an orphan purinoceptor type G-protein coupled receptor, which we refer to as BACH GPCR, as well as homologues, variants or derivatives thereof.

BACH is structurally related to other proteins of the G-protein coupled receptor family, as shown by the results of sequencing the amplified cDNA products encoding human BACH. The cDNA sequence of SEQ ID NO: 1 contains an open reading flame (SEQ ID NO: 2, nucleotide numbers 23 to 800) encoding a polypeptide of 372 amino acids shown in SEQ ID NO: 3. Human BACH is found to map to Homo sapiens chromosome 12p13.3.

### Identities and Similarities to BACH

The amino acid sequence of BACH has about 35 % identity and 57 % similarity (using BLAST) in 309 amino acid residues with human P2Y PURINOCEPTOR 9 (P2Y9) (Accession # U90322.1, Bohm,S.K., Khitin,L.M., Payan,D.P. and Bunnett,N.W., Direct Submission 21-FEB-1997; related accession numbers: NP_005287.1, AAC51301, U66578, AAB62087, U90323, and AAB62088).

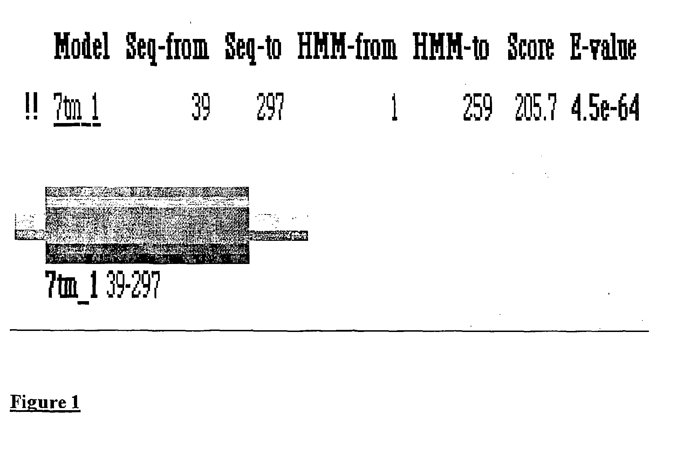

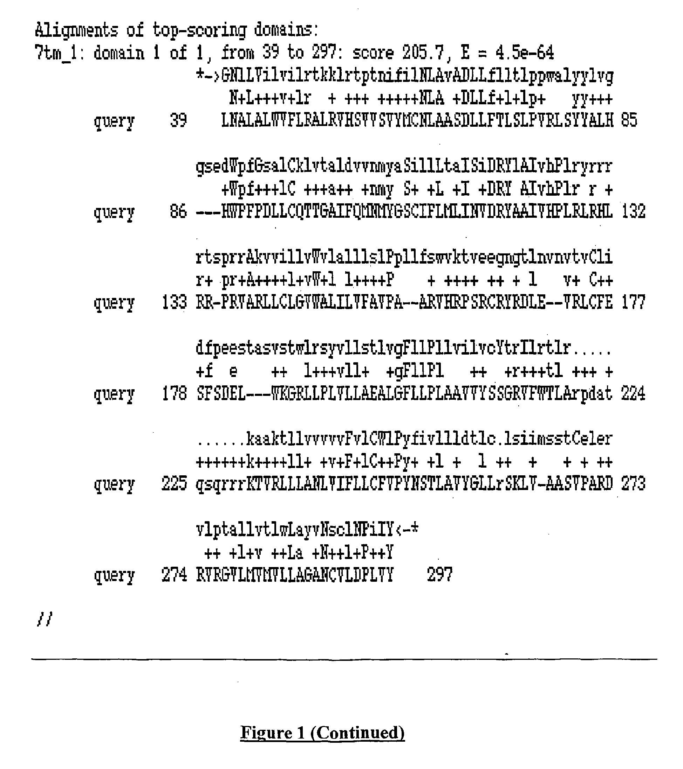

The nucleotide sequence of BACH (SEQ ID NO:1) has about 99% identity (using BLAST) in 672 nucleotide residues with the anonymous Homo sapiens EST testis cDNA from (Accession # AL042117 Ottenwaelder, et al.submitted to Genbank, 29-FEB-2000). Furthermore, BACH (SEQ IDNo: 1) is about 99% identical in 420 nucleotide residues to the anonymous Homo sapiens EST germinal center B cell cDNA clone (Accession # AA769338 NCI-CGAP NO: 1) is also about 98% identical in 159 nucleotides to the anonymous Homo sapiens EST B-cell, chronic lymphotic leukemia cDNA clone (Accession # AI492234 NCI-CGAP e BACH polypeptide (SEQ ID NO: 3) using the HMM structural prediction software of pfam (http://www.sanger.ac.uk/Software/Pfam/search.shtml) confirms that BACH peptide is a GPCR of the 7TM-1 structural class (see Figure 1).

The mouse homologue of the human BACH GPCR has been cloned, and its nucleic acid sequence and amino acid sequence are shown as SEQ ID NO: 4 and SEQ ID NO: 5 respectively. The mouse BACH GPCR cDNA of SEQ ID NO: 4 shows 84% identity with the human BACH GPCR (SEQ ID NO: 2) sequence, while the amino acid sequence (SEQ ID NO: 5) of mouse BACH GPCR shows 80% identity and 85% similarity with human BACH GPCR (SEQ ID NO: 3). A genomic sequence of mouse BACH is also disclosed (SEQ ID NO: 10).

Human and mouse BACH GPCR are therefore members of a large family of G Protein Coupled Receptors (GPCRs).

### Expression Profile of BACH

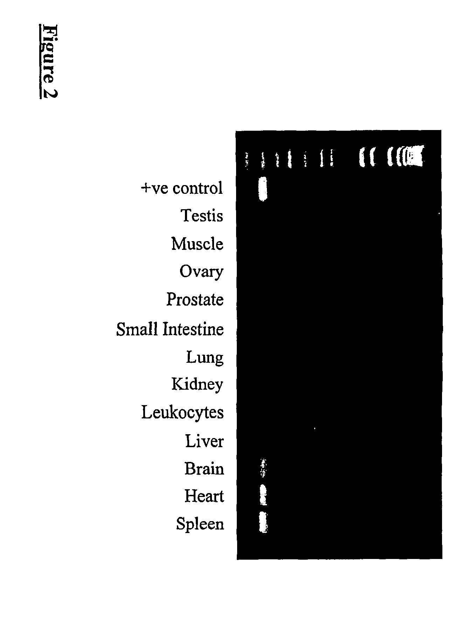

Polymerase chain reaction (PCR) amplification of BACH cDNA detects expression of BACH to varying abundance in human spleen, heart, brain and liver. An expression profile of BACH GPCR is shown in Figure 2. Using BACH cDNA of SEQ ID NO: 1 to search the human EST data sources by BLASTN, identities are found in cDNA derived from libraries originating from B-cells from chronic lymphotic leukemia (Accession # AI492234), germinal center B cell (Accession # AA769338) and testis (Accession # AL042117). This indicates that BACH is expressed in these normal or abnormal tissues. Accordingly, the BACH polypeptides, nucleic acids, probes, antibodies, expression vectors and ligands are useful for detection, diagnosis, treatment and other assays for diseases associated with over-, under- and abnormal expression of BACH GPCR in these and other tissues.

As shown in the Examples, expression data for BACH may also be obtained by use of a knock out mouse for BACH, in which a lacZ reporter gene is integrated into the endogenous BACH gene, and staining for expression of β-galactocidase. Sites of endogenous BACH promoter activity drive expression of the reporter and are visualised, for example histochemically, resulting in blue cells in sites of expression. This pattern faithfully represents the expression pattern of the endogenous BACH transcripts. Staining patterns of lacZ showing the expression of BACH are shown in the Figures.

The above lacZ reporter staining, Northern blot analysis and RT-PCR experiments show that BACH is expressed in the following tissues: Brain, more specifically surface of the cerebellum; Spinal column, more specifically Substancia gelatinosa (caudal/sacral areas); Dorsal root ganglia, more specifically neurones of the A-5 fibre and C fibre class; Trigeminal ganglion and trigeminal nucleus and Cranial nerve 8; Eye, more specifically cells of the conjuctiva; Urinary bladder; Gall bladder; Tongue; Skin, particularly around hair follicles and in the nasal region; pleura and surface of lungs; Salivary glands, regions of submaxillary salivary glands; Gut , more specifically oesophagus, stomach, villi of the small intestine, colon and rectum (crypts); and Fat and pericardium surrounding the heart.

### METHODS EMPLOYED

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning. A Laboratory Manual, Second Edition, Books 1-3, Cold Spring Harbor Laboratory Press; Ausubel, F. M. et al. (1995 and periodic supplements; Current Protocols in Molecular Biology, ch. 9,13, and 16, John Wiley & Sons, New York, N. Y.); B. Roe, J. Crabtree, and A. Kahn, 1996, DNA Isolation and Sequencing : Essential Techniques, John Wiley & Sons; J. M. Polak and James O'D. McGee, 1990, In Situ Hybridization : Principles and Practice ; Oxford University Press ; M. J. Gait (Editor), 1984, Oligonucleotide Synthesis : A Practical Approach, Irl Press; and, D. M. J. Lilley and J. E. Dahlberg, 1992, Methods of Enzymology : DNA Structure Part A : Synthesis and Physical Analysis of DNA Methods in Enzymology, Academic Press.

### BACH GPCR POLYPEPTIDES

As used here, the term"BACH GPCR polypeptide"is intended to refer to a polypeptide comprising the amino acid sequence shown in SEQ ID No. 3 or SEQ ID NO: 5, or a functionally equivalent sequence having at least 90% sequence identity thereto. Preferably, the polypeptide comprises or is a functionally equivalent sequence of the sequence shown in SEQ ID NO: 3.

"Polypeptide"refers to any peptide or protein comprising two or more amino acids joined to each other by peptide bonds or modified peptide bonds, i. e., peptide isosteres. "Polypeptide"refers to both short chains, commonly referred to as peptides, oligopeptides or oligomers, and to longer chains, generally referred to as proteins. Polypeptides may contain amino acids other than the 20 gene-encoded amino acids.

"Polypeptides"include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side- chains and the amino or carboxyl termini. It will be appreciated that the same type of modification may be present in the same or varying degrees at several sites in a given polypeptide. Also, a given polypeptide may contain many types of modifications.

Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-inking, cyclization, disulfide bond formation, demethylation, formation of covalent cross-inks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins-Structure and Molecular Properties, 2nd Ed., T. E. Creighton, W. H. Freeman and Company, New York, 1993 and Wold, F., Posttranslational Protein Modifications : Perspectives and Prospects, pgs. 1-12 in Posttranslational Covalent Modification of Proteins, B. C. Johnson, Ed., Academic Press, New York, 1983; Seifter et al.,"Analysis for protein modifications and nonprotein cofactors", Meth Enzymol (1990) 182: 626-646 and Rattan et aL,"Protein Synthesis: Posttranslational Modifications and Aging", Ann NYAcad Sci (1992) 663: 48-62.

Unless the context admits otherwise, references to "BACH" and "BACH GPCR" include references to the functionally equivalent sequences having at least 90% sequence identity to BACH.

Preferably, as applied to BACH, the resultant amino acid sequence has GPCR activity, more preferably having at least the same activity of the BACH GPCR shown as SEQ ID NO: 3 or SEQ ID NO: 5. Such amino acid sequences also encompass those which are allelic variations of the BACH GPCR nucleic acid sequence.

Where reference is made to the "receptor activity" or "biological activity" of a receptor such as BACH GPCR, these terms are intended to refer to the metabolic or physiological function of the BACH receptor, including similar activities or improved activities or these activities with decreased undesirable side effects. Also included are antigenic and immunogenic activities of the BACH receptor. Examples of GPCR activity, and methods of assaying and quantifying these activities, are known in the art, and are described in detail elsewhere in this document.

As used herein a "deletion" is defined as a change in either nucleotide or amino acid sequence in which one or more nucleotides or amino acid residues, respectively, are absent. As used herein an "insertion" or "addition" is that change in a nucleotide or amino acid sequence which has resulted in the addition of one or more nucleotides or amino acid residues, respectively, as compared to the naturally occurring substance. As used herein "substitution" results from the replacement of one or more nucleotides or amino acids by different nucleotides or amino acids, respectively.

BACH polypeptides used in the methods according to the present invention may also have deletions, insertions or substitutions of amino acid residues which produce a silent change and result in a functionally equivalent amino acid sequence. Deliberate amino acid substitutions may be made on the basis of similarity in polarity, charge, solubility, hydrophobicity, hydrophilicity, and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

BACH polypeptides used in the methods of the invention may further comprise heterologous amino acid sequences, typically at the N-terminus or C-terminus, preferably the N-terminus. Heterologous sequences may include sequences that affect intra or extracellular protein targeting (such as leader sequences). Heterologous sequences may also include sequences that increase the immunogenicity of the polypeptide of the invention and/or which facilitate identification, extraction and/or purification of the polypeptides. Another heterologous sequence that is particularly preferred is a polyamino acid sequence such as polyhistidine which is preferably N-terminal. A polyhistidine sequence of at least 10 amino acids, preferably at least 17 amino acids but fewer than 50 amino acids is especially preferred.

The BACH GPCR polypeptides may be in the form of the "mature" protein or may be a part of a larger protein such as a fusion protein. It is often advantageous to include an additional amino acid sequence which contains secretory or leader sequences, pro- sequences, sequences which aid in purification such as multiple histidine residues, or an additional sequence for stability during recombinant production.

BACH polypeptides used in the methods of the invention are advantageously made by recombinant means, using known techniques. However they may also be made by synthetic means using techniques well known to skilled persons such as solid phase synthesis. Polypeptides of the invention may also be produced as fusion proteins, for example to aid in extraction and purification. Examples of fusion protein partners include glutathione-S-transferase (GST), 6xHis, GAL4 (DNA binding and/or transcriptional activation domains) and β- galactosidase. It may also be convenient to include a proteolytic cleavage site between the fusion protein partner and the protein sequence of interest to allow removal of fusion protein sequences, such as a thrombin cleavage site. Preferably the fusion protein will not hinder the function of the protein of interest sequence.

BACH polypeptides used in the methods of the invention may be in a substantially isolated form. This term is intended to refer to alteration by the hand of man from the natural state. If an "isolated" composition or substance occurs in nature, it has been changed or removed from its original environment, or both. For example, a polynucleotide, nucleic acid or a polypeptide naturally present in a living animal is not "isolated," but the same polynucleotide, nucleic acid or polypeptide separated from the coexisting materials of its natural state is "isolated" , as the term is employed herein.

It will however be understood that the BACH GPCR protein may be mixed with carriers or diluents which will not interfere with the intended purpose of the protein and still be regarded as substantially isolated. A polypeptide of the invention may also be in a substantially purified form, in which case it will generally comprise the protein in a preparation in which more than 90%, for example, 95%, 98% or 99% of the protein in the preparation is a BACH GPCR polypeptide used in the methods of the invention.

The present invention also relates to peptides comprising a portion of a BATCH polypeptide used in the methods according to the invention. Thus, fragments of BACH GPCR are included, provided they are functionally equivalent to the BACH GPCR and have at least 90% sequence identity thereto. The peptide may be derived from a BACH GPCR polypeptide as disclosed here, for example by digestion with a suitable enzyme, such as trypsin. Alternatively the peptide, fragment, etc may be made by recombinant means, or synthesised synthetically.

The term "peptide" includes the various synthetic peptide variations known in the art, such as a retroinverso D peptides. The peptide may be an antigenic determinant and/or a T-cell epitope. The peptide may be immunogenic *in vivo.* Preferably the peptide is capable of inducing neutralising antibodies *in vivo.*

By aligning BACH GPCR sequences from different species, it is possible to determine which regions of the amino acid sequence are conserved between different species ("homologous regions"), and which regions vary between the different species ("heterologous regions").

The BACH polypeptides used in the methods according to the invention may therefore comprise a sequence which corresponds to at least part of a homologous region. A homologous region shows a high degree of homology between at least two species. For example, the homologous region may show at least 70%, preferably at least 80%, more preferably at least 90%, even more preferably at least 95% identity at the amino acid level using the tests described above. Peptides which comprise a sequence which corresponds to a homologous region may be used in therapeutic strategies as explained in further detail below. Alternatively, the BACH GPCR peptide may comprise a sequence which corresponds to at least part of a heterologous region. A heterologous region shows a low degree of homology between at least two species.

### BACH GPCR POLYNUCLEOTIDES AND NUCLEIC ACIDS

This invention encompasses BACH polynucleotides, BACH nucleotides and BACII nucleic acids, methods of production, uses of these, etc, as described in further detail elsewhere in this document.

The terms "BACH polynucleotide", "BACH nucleotide" and "BACH nucleic acid" may be used interchangeably, and are intended to refer to a polynucleotide/nucleic acid comprising a nucleic acid sequence as shown in SEQ ID NO: 1, SEQ ID NO : 2, SEQ ID NO: 4, SEQ ID NO: 10, or a functionally equivalent sequence having at least 90% sequence identity thereto. Preferably, the polynucleotide/nucleic acid comprises or is a functionally equivalent sequence having at least 90% sequence identity to the nucleic acid sequence SEQ ID NO: 1 or SEQ ID NO: 2, most preferably, SEQ ID NO: 2. The terms"BACH polynucleotide","BACH nucleotide"and"BACH nucleic acid"should be understood to specifically include both cDNA and genomic BACH sequences, for example, the mouse BACH genomic sequence shown below (SEQ ID NO: 10).

These terms are also intended to include a nucleic acid sequence capable of encoding a polypeptides and/or a peptide of the present invention, i. e., a BACH polypeptide. Thus, BACH GPCR polynucleotides and nucleic acids comprise a nucleotide sequence capable of encoding a polypeptide comprising the amino acid sequence shown in SEQ ID NO: 3 or SEQ ID NO: 5, or a functionally equivalent sequence having at least 90% sequence identity thereto.
Preferably, the BACH GPCR polynucleotides and nucleic acids comprise a nucleotide sequence capable of encoding a polypeptide comprising the amino acid sequence shown in SEQ ID NO: 3, or a functionally equivalent sequence having at least 90% sequence identity thereto.

"Polynucleotide" generally refers to any polyribonucleotide or polydeoxribonucleotide, which may be unmodified RNA or DNA or modified RNA or DNA."Polynucleotides"include, without limitation single-and double-stranded DNA, DNA that is a mixture of single-and double-stranded regions, single-and double-stranded RNA, and RNA that is mixture of single-and double-stranded regions, hybrid molecules comprising DNA and RNA that may be single-stranded or, more typically, double- stranded or a mixture of single-and double-stranded regions. In addition,"polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons.

"Modified"bases include, for example, tritylated bases and unusual bases such as inosine. A variety of modifications has been made to DNA and RNA; thus,"polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

It will be understood by the skilled person that numerous nucleotide sequences can encode the same polypeptide as a result of the degeneracy of the genetic code.

As used herein, the term "nucleotide sequence" refers to nucleotide sequences, oligonucleotide sequences, polynucleotide sequences and variants, homologues, fragments and derivatives thereof (such as portions thereof). The nucleotide sequence may be DNA or RNA of genomic or synthetic or recombinant origin which may be double-stranded or single-stranded whether representing the sense or antisense strand or combinations thereof.
The term nucleotide sequence may be prepared by use of recombinant DNA techniques (for example, recombinant DNA).

Preferably, the term"nucleotide sequence"means DNA.

The terms "functionally equivalent sequence" in relation to the present invention include any substitution of, variation of, modification of, replacement of, deletion of or addition of one (or more) nucleic acids from or to the sequence of a BACH nucleotide sequence. Unless the context admits otherwise, references to "BACH" and "BACH GPCR" include references to such functionally equivalent sequences of BACH.

Preferably, the resultant nucleotide sequence encodes a polypeptide having at least the same activity of the GPCR shown as SEQ ID NO: 3 or SEQ ID NO : 5.

### BACH Control Regions

For some purposes, it may be necessary to utilise or investigate control regions of BACH. Such control regions include promoters, enhancers and locus control regions. By a control region we mean a nucleic acid sequence or structure which is capable of modulating the expression of a coding sequence which is operatively linked to it.

For example, control regions are useful in generating transgenic animals expressing BACH. Furthermore, control regions may be used to generate expression constructs for BACH. Control regions from different individuals in a population may be sequenced to identify non-coding polymorphisms, which may affect the expression level of BACH. This is described in further detail below.

Identification of control regions of BACH is straightforward, and may be carried out in a number of ways. For example, the coding sequence of BACH may be obtained from an organism, by screening a cDNA library using a human or mouse BACH cDNA sequence as a probe. 5' sequences may be obtained by screening an appropriate genomic library, or by primer extension as known in the art. Database searching of genome databases may also be employed. Such 5' sequences which are particularly of interest include non-coding regions. The 5' regions may be examined by eye, or with the aid of computer programs, to identify sequence motifs which indicate the presence of promoter and/or enhancer regions.

Furthermore, sequence alignments may be conducted of BACH nucleic acid sequences from two or more organisms. By aligning BACH GPCR sequences from different species, it is possible to determine which regions of the amino acid sequence are conserved between different species. Such conserved regions are likely to contain control regions for the gene in question (i.e., BACH). The mouse and human genomic sequences as disclosed here, for example, a mouse BACH genomic sequence (SEQ ID NO: 10), may be employed for this purpose. Furthermore, BACH homologues from other organisms may be obtained using standard methods of screening using appropriate probes generated from the mouse and human BACH sequences. The genome of the pufferfish (*Takifugu rubripes*) may also be screened to identify a BACH homologue; comparison of the 5' non-coding region of the Fugu BACH gene with a mouse or human genomic BACH sequence (e.g., SEQ ID NO: 10, mouse BACH genomic sequence) may be used to identify conserved regions containing control regions.

Deletion studies may also be conducted to identify promoter and/or enhancer regions for BACH.

The identity of putative control regions may be confirmed by molecular biology experiments, in which the candidate sequences are linked to a reporter gene and the expression of the reporter detected.

### BACH GPCR ASSOCIATED DISEASES

According to the methods described here, BACH GPCR is useful for treating and diagnosing a range of diseases.

We demonstrate here that human BACH maps to Homo sapiens chromosome 12p13. 3. Accordingly, in a specific embodiment, BACH, and agents which bind to, agonise or antagonise BACH may be used to treat or diagnose a disease which maps to this locus, chromosomal band, region, arm or the same chromosome.

Furthermore, we demonstrate an expression pattern of BACH in transgenic mice, and identify the phenotypes of such mice. These indicate a role for BACH in sensing pain, maintaining balance and in secretion. BACH and agents which bind to, agonise or antagonise BACH may be used to treat or diagnose a disease in which there is a disorder in any of these.

Accordingly, in one embodiment of the invention, BACH GPCR may be used to diagnose, by any means as described in this document, pain or a disorder of urogenital function.

In particular, we specifically envisage the use of nucleic acids, vectors comprising BACH GPCR nucleic acids, polypeptides, including functionally equivalent sequences having at least 90% sequence identity thereto, pharmaceutical compositions, host cells, and transgenic animals comprising BACH GPCR nucleic acids and/or polypeptides, for the treatment or diagnosis of the specific diseases listed above. Furthermore, we envisage the use of compounds capable of interacting with or binding to BACH GPCR, preferably antagonists of a BACH GPCR, preferably a compound capable of lowering the endogenous level of cyclic AMP in a cell, antibodies BACH BACH GPCR, as well as methods of making or identifying these, in diagnosis or treatment of the specific diseases mentioned above.

Methods of linkage mapping to identify such or further specific diseases treatable or diagnosable by use of BACH GPCR are known in the art, and are also described elsewhere in this document.

### PAIN AND SENSITIVITY

As shown in the Examples and Figures, BACH is expressed in the nervous system of mice. Altogether, the presence of LacZ staining in the Dorsal root ganglia, the spinal cord and the trigeminal ganglion and trigeminal nucleus indicates a very strong potential role for BACH in pain and sensitivity (Julius and Basbaum, 2001¹).

This is consistent with the lower response of the BACH mutant to the paw-pressure test the hypoalgesic responses seen in other tests such as the tail flick test. Similarly, the expression in the viscera such as in the tubes of the digestive system (oesphagus, gut, stomach) and sacs such as the bladder and gall bladder, lungs, salivary glands and eyes is strongly reminiscent of the expression pattern of P2X3 which is involved in "stretching" or visceral pain sensitivity in these organs and as well as neuropathic/inflammatory pain (Burnstock 2001, Cockayne et al 2000, Souslova et al, 2000).

Examination of the expression pattern and analgesia phenotypic data of the mutant mice lacking BACH therefore show that BACH is therefore a target for potential treatment of trigeminal neuralgia as well as migraine. Accordingly, therapeutic agents developed using the methods and compositions described here may be used as analgesics. Such therapeutic agents may comprise agonists or antagonists of BACH, preferably antagonists of BACH.

The agents identified may be used in the treatment and management of neuropathic, inflammatory and visceral pain. These analgesic type therapeutics may among other conditions be used to treat Trigeminal neuralgia, orofacial pain, pain associated with toothache, irritable bowel syndrome, Barrett's oesophagus, glaucoma, pain associated with cancer, diabetic neuropathies, Herpes infections, HIV infections, migraine and skin sensitivity associated with migraine, allodynia, toothache, neuroma (whether caused by amputation, nerve transaction or trauma), nerve compression (caused by tumours, entrapment or crush) and pain due to damage of the spinal cord or brain.

Orofacial pain is a consequence of trigeminal neuralgia, in which paroxysmal pain radiates over one, or two divisions of the trigeminal nerve. The opthalmic division is rarely affected. Drug treatment is usually effective but if it fails surgical treatment is used. None of these surgical treatments has proved satisfactory. No specific drug has been developped yet.

Skin sensitivity appears among the majority of migraine sufferers. Burstein et al published a study showing that 79 percent of 44 migraine patients had extreme skin sensitivity. Burstein describes the extreme effects of migraine sufferers are unable to undertake day to day tasks such as brushing hair, wearrings or eyeglasses, or shaving beards because of the extreme pain.

In migraine series of neuronal clusters --in the sensory ganglions, the brainstem and the thalamus --become sensitised in a kind of domino effect. If the sensitised cluster, a group of nerve cells that acts like the hub of a computer network, happens to be connected to the skin, the result can be skin sensitivity. The problem starts with the release of inflammatory substances from the dura, and from blood vessels and nerve endings in the brain. This oversensitizes the trigeminal ganglion. When oversensitized, the ganglion interprets normal pressure inside the skull as the throbbing pain of migraine. Because the trigeminal ganglion seems to cause the primary pain of migraine, it is the target of current migraine drugs, which block serotonin receptors in sensory neurons connected to the dura. The drugs are often effective, but only if taken immediately after the headache begins.

The oversensitised trigeminal ganglion may, in turn, send signals to the nucleus caudalis, at the top of the spinal cord. Unlike the trigeminal ganglion, this group of nerves is connected to the skin, particularly near the eye, where the most dramatic skin sensitivity is found in migraine sufferers. In rats, once the trigeminal ganglion has activated the nucleus caudalis for an hour, the nucleus caudalis remains overwrought even if the trigeminal ganglion is calmed by drugs--as existing migraine treatments often do. The experiment also indicates that hyper-sensitive neurons in the nucleus caudalis interpret soft touches on the skin as pain. Although current migraine drugs often work if taken quickly after the headache's onset, that is impossible for people who don't have drugs handy or get the headaches while asleep. According to Burnstein, this could explain why current anti- migraine therapies, which work on the primary cluster, are only effective if taken during the firsts hour after an attack has begun. An important target therefore comprises secondary neurons.

The Burnstein study therefore shows that skin sensitivity has a clear origin in the hypervigilance of oversensitized nerve cells. (Burstein, R. et al, 2000 a¹) and b¹)

### CALCULATION OF SEQUENCE HOMOLOGY

Sequence identity with respect to any of the sequences presented here can be determined by a simple "eyeball" comparison (i.e. a strict comparison) of any one or more of the sequences with another sequence to see if that other sequence has, for example, at least 70% sequence identity to the sequence(s).

Relative sequence identity can also be determined by commercially available computer programs that can calculate % identity between two or more sequences using any suitable algorithm for determining identity, using for example default parameters. A typical example of such a computer program is CLUSTAL. Other computer program methods to determine identify and similarity between the two sequences include but are not limited to the GCG program package (Devereux et al 1984 Nucleic Acids Research 12: 387) and FASTA (Atschul et al 1990 J Molec Biol 403-410).

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is-12 for a gap and-4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U. S. A.; Devereux et al., 1984, Nucleic Acids Research 12: 387). Examples of other software than can perform sequence comparisons include, but are not limited to, the BLAST package (Ausubel et al., 1999 ibid-Chapter 18), FASTA (Atschul et al., 1990, J. Mol. Biol., 403-410) and the GENEWORKS suite of comparison tools. Both BLAST and FASTA are available for offline and online searching (Ausubel et al., 1999 ibid, pages 7- 58 to 7-60).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix-the default matrix for the BLAST suite of programs. GCG Wisconsin programs generally use either the public default values or a custom symbol comparison table if supplied. It is preferred to use the public default values for the GCG package, or in the case of other software, the default matrix, such as BLOSUM62.

Advantageously, the BLAST algorithm is employed, with parameters set to default values. The BLAST algorithm is described in detail at http://www.ncbi.nih. gov/BLAST/blast_help.html. The search parameters are defined as follows, can be advantageously set to the defined default parameters.

Advantageously, "substantial identity" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (Karlin and Altschul 1990, Proc. Natl. Acad Sci. USA 87:2264-68; Karlin and Altschul, 1993, Proc. Natl. Acad. Sci. USA 90:5873-7; see http://www.ncbi.nih.govBLAST/blast_help.html) with a few enhancements. The BLAST programs are tailored for sequence similarity searching, for example to identify homologues to a query sequence. For a discussion of basic issues in similarity searching of sequence databases, see Altschul et al (1994) Nature Genetics 6:119-129.

The five BLAST programs available at http://www.ncbi.nlm.nih.gov perform the following tasks: **blastp -** compares an amino acid query sequence against a protein sequence database; **blastn -** compares a nucleotide query sequence against a nucleotide sequence database; **blastx -** compares the six-frame conceptual translation products of a nucleotide query sequence (both strands) against a protein sequence database; **tblastn-**compares a protein query sequence against a nucleotide sequence database dynamically translated in all six reading frames (both strands); **tblastx -** compares the six-frame translations of a nucleotide query sequence against the six-frame translations of a nucleotide sequence database.

BLAST uses the following search parameters:
HISTOGRAM - Display a histogram of scores for each search; default is yes. (See parameter H in the BLAST Manual).
DESCRIPTIONS - Restricts the number of short descriptions of matching sequences reported to the number specified; default limit is 100 descriptions. (See parameter V in the manual page).
EXPECT - The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).
CUTOFF - Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.
ALIGNMENTS - Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).
MATRIX - Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.
STRAND - Restrict a TBLASTN search to just the top or bottom strand of the database sequences; or restrict a BLASTN, BLASTX or TBLASTX search to just reading frames on the top or bottom strand of the query sequence.
FILTER - Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi - Causes NCBI gi identifiers to be shown in the output, in addition to the accession and/or locus name.

Most preferably, sequence comparisons are conducted using the simple BLAST search algorithm provided at http://www.ncbi.nlm.nih.gov/BLAST. In some embodiments of the present invention, no gap penalties are used when determining sequence identity.

### CLONING OF BACH GPCR AND HOMOLOGUES

The present invention also encompasses nucleotide sequences that are complementary to the sequences presented here, or any fragment or derivative thereof. If the sequence is complementary to a fragment thereof then that sequence can be used as a probe to identify and clone similar GPCR sequences in other organisms etc.

The present invention thus enables the cloning of BACH GPCR, its homologues and other structurally or functionally related genes from human and other species such as mouse, pig, sheep, etc to be accomplished. Polynucleotides of the invention, which are identical or sufficiently identical to a nucleotide sequence contained in SEQ ID NO: 1, SEQ ID NO: 2, SEQ ID NO: 4 or a mouse BACH genomic sequence (SEQ ID NO: 10), or a fragment thereof, may be used as hybridization probes for cDNA and genomic DNA as appropriate, to isolate partial or full-length cDNAs and genomic clones encoding BACH GPCR from appropriate libraries. Such probes may also be used to isolate cDNA and genomic clones of other genes (including genes encoding homologues and orthologues from species other than human) that have sequence similarity, preferably high sequence similarity, to the BACH GPCR gene. Hybridization screening, cloning and sequencing techniques are known to those of skill in the art and are described in, for example, Sambrook et al (*supra*).

Typically nucleotide sequences suitable for use as probes are 70% identical, preferably 80% identical, more preferably 90% identical, even more preferably 95% identical to that of the referent. The probes generally will comprise at least 15 nucleotides. Preferably, such probes will have at least 30 nucleotides and may have at least 50 nucleotides. Particularly preferred probes will range between 150 and 500 nucleotides, more particularly about 300 nucleotides.

In one embodiment, to obtain a polynucleotide encoding a BACH GPCR polypeptide, including homologues and orthologues from species other than human, comprises the steps of screening an appropriate library under stringent hybridization conditions with a labelled probe having the SEQ ID NO: 1, SEQ ID NO: 2, SEQ ID NO: 4 or a mouse BACH genomic sequence (SEQ ID NO: 10), or a fragment thereof and isolating partial or full-length cDNA and genomic clones containing said polynucleotide sequence. Such hybridization techniques are well known to those of skill in the art. Stringent hybridization conditions are as defined above or alternatively conditions under overnight incubation at 42 degrees C. in a solution comprising: 50% formamide, 5XSSC (150 mM NaCl, 15mM trisodium citrate), 50 mM sodium phosphate (pH7.6), 5XDenhardt's solution, 10% dextran sulphate, and 20 microgram/ml denatured, sheared salmon sperm DNA, followed by washing the filters in 0.1XSSC at about 65 degrees C.

### Functional Assay for BACH GPCR

The cloned putative BACH GPCR polynucleotides may be verified by sequence analysis or functional assays. For example, the putative BACH GPCR or homologue may be assayed for receptor activity as follows. Capped RNA transcripts from linearized plasmid templates encoding the BACH receptor cDNAs of the invention are synthesized *in vitro* with RNA polymerases in accordance with standard procedures. *In vitro* transcripts are suspended in water at a final concentration of 0.2 mg/ml. Ovarian lobes are removed from adult female toads, Stage V defolliculated oocytes are obtained, and RNA transcripts (10 ng/oocyte) are injected in a 50 nl bolus using a microinjection apparatus. Two electrode voltage clamps are used to measure the currents from individual *Xenopus* oocytes in response to agonist exposure. Recordings are made in Ca²⁺ free Barth's medium at room temperature. The *Xenopus* system may also be used to screen known ligands and tissue/cell extracts for activating ligands, as described in further detail below.

### Expression Assays for BACH GPCR

In order to design useful therapeutics for treating BACH GPCR associated diseases, it is useful to determine the expression profile of BACH (whether wild-type or a particular mutant). Thus, methods known in the art may be used to determine the organs, tissues and cell types (as well as the developmental stages) in which BACH is expressed.
For example, traditional or "electronic" Northerns may be conducted. Reverse- transcriptase PCR (RT-PCR) may also be employed to assay expression of the BACH gene or mutant. More sensitive methods for determining the expression profile of BACH include RNAse protection assays, as known in the art.

Northern analysis is a laboratory technique used to detect the presence of a transcript of a gene and involves the hybridization of a labeled nucleotide sequence to a membrane on which RNAs from a particular cell type or tissue have been bound.
(Sambrook, supra, ch. 7 and Ausubel, F. M. et al. supra, ch. 4 and 16.) Analogous computer techniques ("electronic Northerns") applying BLAST may be used to search for identical or related molecules in nucleotide databases such as GenBank or the LIFESEQ database (Incyte Pharmaceuticals). This type of analysis has advantages in that they may be faster than multiple membrane-based hybridizations. In addition, the sensitivity of the computer search can be modified to determine whether any particular match is categorized as exact or homologous.

The polynucleotides and polypeptides of the present invention, including the probes described above, may be employed as research reagents and materials for discovery of treatments and diagnostics to animal and human disease, as explained in further detail elsewhere in this document.

### EXPRESSION OF BACH GPCR POLYPEPTIDES

In order to express a biologically active BACH GPCR, the nucleotide sequences encoding BACH GPCR or functional equivalents thereof are inserted into appropriate expression vector, i.e., a vector which contains the necessary elements for the transcription and translation of the inserted coding sequence.

Methods which are well known to those skilled in the art are used to construct expression vectors containing sequences encoding BACH GPCR and appropriate transcriptional and translational control elements. These methods include *in vitro* recombinant DNA techniques, synthetic techniques, and in vivo genetic recombination. Such techniques are described in Sambrook, J. et al. (1989; Molecular Cloning, A Laboratory Manual, ch. 4, 8, and 16-17, Cold Spring Harbor Press, Plainview, N.Y.) and Ausubel, F. M. et al. (1995 and periodic supplements; Current Protocols in Molecular Biology, ch. 9, 13, and 16, John Wiley & Sons, New York, N.Y.).

A variety of expression vector/host systems may be utilized to contain and express sequences encoding BACH GPCR. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus (CaMV) or tobacco mosaic virus (TMV)) or with bacterial expression vectors (e.g., Ti or pBR322 plasmids); or animal cell systems. The invention is not limited by the host cell employed.

The "control elements" or "regulatory sequences" are those non-translated regions of the vector (i.e., enhancers, promoters, and 5' and 3' untranslated regions) which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the BLUESCRIPT phagemid (Stratagene, La Jolla, Calif.) or PSPORT1 plasmid (GIBCO/BRL), and the like, may be used. The baculovirus polyhedrin promoter may be used in insect cells. Promoters or enhancers derived from the genomes of plant cells (e.g., heat shock, RUBISCO, and storage protein genes) or from plant viruses (e.g., viral promoters or leader sequences) may be cloned into the vector. In mammalian cell systems, promoters from mammalian genes or from mammalian viruses are preferable. If it is necessary to generate a cell line that contains multiple copies of the sequence encoding BACH GPCR, vectors based on SV40 or EBV may be used with an appropriate selectable marker.

In bacterial systems, a number of expression vectors may be selected depending upon the use intended for BACH GPCR. For example, when large quantities of BACH GPCR are needed for the induction of antibodies, vectors which direct high level expression of fusion proteins that are readily purified may be used. Such vectors include, but are not limited to, multifunctional E. coli cloning and expression vectors such as BLUESCRIPT (Stratagene), in which the sequence encoding BACH GPCR may be ligated into the vector in frame with sequences for the amino-terminal Met and the subsequent 7 residues of β-galactosidase so that a hybrid protein is produced, pIN vectors (Van Heeke, G. and S. M. Schuster (1989) J. Biol. Chem. 264:5503-5509), and the like. pGEX vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast *Saccharomyces cerevisiae,* a number of vectors containing constitutive or inducible promoters, such as alpha factor, alcohol oxidase, and PGH, may be used. For reviews, see Ausubel (supra) and Grant et al. (1987; Methods Enzymol. 153:516-544).

In cases where plant expression vectors are used, the expression of sequences encoding BACH GPCR may be driven by any of a number of promoters. For example, viral promoters such as the 35S and 19S promoters of CaMV may be used alone or in combination with the omega leader sequence from TMV. (Takamatsu, N. (1987) EMBO J. 6:307-311.) Alternatively, plant promoters such as the small subunit of RUBISCO or heat shock promoters may be used. (Coruzzi, G. et al. (1984) EMBO J. 3:1671-1680; Broglie, R. et al. (1984) Science 224:838-843; and Winter, J. et al. (1991) Results Probl. Cell Differ. 17:85-105.) These constructs can be introduced into plant cells by direct DNA transformation or pathogen-mediated transfection. Such techniques are described in a number of generally available reviews. (See, for example, Hobbs, S. or Murry, L. E. in McGraw Hill Yearbook of Science and Technology (1992) McGraw Hill, New York, N.Y.; pp. 191-196.).

An insect system may also be used to express BACH GPCR. For example, in one such system, *Autographa californica* nuclear polyhedrosis virus (AcNPV) is used as a vector to express foreign genes in *Spadaptera frugiperda* cells or in *Trichoplusia* larvae. The sequences encoding BACH GPCR may be cloned into a non-essential region of the virus, such as the polyhedrin gene, and placed under control of the polyhedrin promoter. Successful insertion of BACH GPCR will render the polyhedrin gene inactive and produce recombinant virus lacking coat protein. The recombinant viruses may then be used to infect, for example, *S. frugiperda* cells or *Trichoplusia* larvae in which BACH GPCR may be expressed. (Engelhard, E. K. et al. (1994) Proc. Nat. Acad. Sci. 91:3224-3227.)

In mammalian host cells, a number of viral-based expression systems may be utilized. In cases where an adenovirus is used as an expression vector, sequences encoding BACH GPCR may be ligated into an adenovirus transcription/translation complex consisting of the late promoter and tripartite leader sequence. Insertion in a non-essential E1 or E3 region of the viral genome may be used to obtain a viable virus which is capable of expressing BACH GPCR in infected host cells. (Logan, J. and T. Shenk (1984) Proc. Natl. Acad. Sci. 81:3655-3659.) In addition, transcription enhancers, such as the Rous sarcoma virus (RSV) enhancer, may be used to increase expression in mammalian host cells.

Thus, for example, the BACH receptors of the present invention are expressed in either human embryonic kidney 293 (HEK293) cells or adherent dhfr CHO cells. To maximize receptor expression, typically all 5' and 3' untranslated regions (UTRs) are removed from the receptor cDNA prior to insertion into a pCDN or pCDNA3 vector. The cells are transfected with individual receptor cDNAs by lipofectin and selected in the presence of 400 mg/ml G418. After 3 weeks of selection, individual clones are picked and expanded for further analysis. HEK293 or CHO cells transfected with the vector alone serve as negative controls. To isolate cell lines stably expressing the individual receptors, about 24 clones are typically selected and analyzed by Northern blot analysis. Receptor mRNAs are generally detectable in about 50% of the G418-resistant clones analyzed.

Human artificial chromosomes (HACs) may also be employed to deliver larger fragments of DNA than can be contained and expressed in a plasmid. HACs of about 6 kb to 10 Mb are constructed and delivered via conventional delivery methods (liposomes, polycationic amino polymers, or vesicles) for therapeutic purposes.

Specific initiation signals may also be used to achieve more efficient translation of sequences encoding BACH GPCR. Such signals include the ATG initiation codon and adjacent sequences. In cases where sequences encoding BACH GPCR and its initiation codon and upstream sequences are inserted into the appropriate expression vector, no additional transcriptional or translational control signals may be needed. However, in cases where only coding sequence, or a fragment thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers appropriate for the particular cell system used, such as those described in the literature. (Scharf, D. et al. (1994) Results Probl. Cell Differ. 20:125-162.)

In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding, and/or function. Different host cells which have specific cellular machinery and characteristic mechanisms for post-translational activities (e.g., CHO, HeLa, MDCK, HEK293, and WI38), are available from the American Type Culture Collection (ATCC, Bethesda, Md.) and may be chosen to ensure the correct modification and processing of the foreign protein.

For long term, high yield production of recombinant proteins, stable expression is preferred. For example, cell lines capable of stably expressing BACH GPCR can be transformed using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase genes (Wigler, M. et al. (1977) Cell 11:223-32) and adenine phosphoribosyltransferase genes (Lowy, I. et al. (1980) Cell 22:817-23), which can be employed in tk- or apr cells, respectively. Also, antimetabolite, antibiotic, or herbicide resistance can be used as the basis for selection. For example, dhfr confers resistance to methotrexate (Wigler, M. et al. (1980) Proc. Natl. Acad. Sci. 77:3567-70); npt confers resistance to the aminoglycosides neomycin and G-418 (Colbere-Garapin, F. et al (1981) J. Mol. Biol. 150:1-14); and als or pat confer resistance to chlorsulfuron and phosphinotricin acetyltransferase, respectively (Murry, supra). Additional selectable genes have been described, for example, trpB, which allows cells to utilize indole in place of tryptophan, or hisD, which allows cells to utilize histinol in place of histidine. (Hartman, S. C. and R. C. Mulligan (1988) Proc. Natl. Acad. Sci. 85:8047-51.) Recently, the use of visible markers has gained popularity with such markers as anthocyanins, β-glucuronidase and its substrate GUS, and luciferase and its substrate luciferin. These markers can be used not only to identify transformants, but also to quantify the amount of transient or stable protein expression attributable to a specific vector system. (Rhodes, C. A. et al. (1995) Methods Mol. Biol. 55:121-131.)

Although the presence/absence of marker gene expression suggests that the gene of interest is also present, the presence and expression of the gene may need to be confirmed. For example, if the sequence encoding BACH GPCR is inserted within a marker gene sequence, transformed cells containing sequences encoding BACH GPCR can be identified by the absence of marker gene function. Alternatively, a marker gene can be placed in tandem with a sequence encoding BACH GPCR under the control of a single promoter. Expression of the marker gene in response to induction or selection usually indicates expression of the tandem gene as well.

Alternatively, host cells which contain the nucleic acid sequence encoding BACH GPCR and express BACH GPCR may be identified by a variety of procedures known to those of skill in the art. These procedures include, but are not limited to, DNA--DNA or DNA-RNA hybridizations and protein bioassay or immunoassay techniques which include membrane, solution, or chip based technologies for the detection and/or quantification of nucleic acid or protein sequences.

The presence of polynucleotide sequences encoding BACH GPCR can be detected by DNA--DNA or DNA-RNA hybridization or amplification using probes or fragments or fragments of polynucleotides encoding BACH GPCR. Nucleic acid amplification based assays involve the use of oligonucleotides or oligomers based on the sequences encoding BACH GPCR to detect transformants containing DNA or RNA encoding BACH GPCR.

A variety of protocols for detecting and measuring the expression of BACH GPCR, using either polyclonal or monoclonal antibodies specific for the protein, are known in the art. Examples of such techniques include enzyme-linked immunosorbent assays (ELISAs), radioimmunoassays (RIAs), and fluorescence activated cell sorting (FACS). A two-site, monoclonal-based immunoassay utilizing monoclonal antibodies reactive to two non-interfering epitopes on BACH GPCR is preferred, but a competitive binding assay may be employed. These and other assays are well described in the art, for example, in Hampton, R. et al. (1990; Serological Methods, a Laboratory Manual, Section IV, APS Press, St Paul, Minn.) and in Maddox, D. E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides encoding BACH GPCR include oligolabeling, nick translation, end-labeling, or PCR amplification using a labeled nucleotide. Alternatively, the sequences encoding BACH GPCR, or any fragments thereof, may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits, such as those provided by Pharmacia & Upjohn (Kalamazoo, Mich.), Promega (Madison, Wis.), and U.S. Biochemical Corp. (Cleveland, Ohio). Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding BACH GPCR may be cultured under conditions suitable for the expression and recovery of the protein from cell culture. The protein produced by a transformed cell may be located in the cell membrane, secreted or contained intracellularly depending on the sequence and/or the vector used. As will be understood by those of skill in the art, expression vectors containing polynucleotides which encode BACH GPCR may be designed to contain signal sequences which direct secretion of BACH GPCR through a prokaryotic or eukaryotic cell membrane. Other constructions may be used to join sequences encoding BACH GPCR to nucleotide sequences encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). The inclusion of cleavable linker sequences, such as those specific for Factor XA or enterokinase (Invitrogen, San Diego, Calif.), between the purification domain and the BACH GPCR encoding sequence may be used to facilitate purification. One such expression vector provides for expression of a fusion protein containing BACH GPCR and a nucleic acid encoding 6 histidine residues preceding a thioredoxin or an enterokinase cleavage site. The histidine residues facilitate purification on immobilized metal ion affinity chromatography (IMIAC; described in Porath, J. et al. (1992) Prot. Exp. Purif. 3: 263-281), while the enterokinase cleavage site provides a means for purifying BACH GPCR from the fusion protein. A discussion of vectors which contain fusion proteins is provided in Kroll, D. J. et al. (1993; DNA Cell Biol. 12:441-453).

Fragments of BACH GPCR may be produced not only by recombinant production, but also by direct peptide synthesis using solid-phase techniques. (Merrifield J. (1963) J. Am. Chem. Soc. 85:2149-2154.) Protein synthesis may be performed by manual techniques or by automation. Automated synthesis may be achieved, for example, using the Applied Biosystems 431A peptide synthesizer (Perkin Elmer). Various fragments of BACH GPCR may be synthesized separately and then combined to produce the full length molecule.

### BIOSENSORS

The BACH polypeptides, nucleic acids, probes, antibodies, expression vectors and ligands are useful as (and for the production of) biosensors.

According to Aizawa (1988), Anal. Chem. Symp. 17: 683, a biosensor is defined as being a unique combination of a receptor for molecular recognition, for example a selective layer with immobilized antibodies or receptors such as a BACH G-protein coupled receptor, and a transducer for transmitting the values measured. One group of such biosensors will detect the change which is caused in the optical properties of a surface layer due to the interaction of the receptor with the surrounding medium. Among such techniques may be mentioned especially ellipso-metry and surface plasmon resonance. Biosensors incorporating BACH may be used to detect the presence or level of BACH ligands, for example, nucleotides such as purines or purine analogues, or analogues of these ligands. The construction of such biosensors is well known in the art.

Thus, cell lines expressing BACH receptor may be used as reporter systems for detection of ligands such as ATP via receptor-promoted formation of [3H] inositol phosphates or other second messengers (Watt et al., 1998, JBiol Chena May 29 ; 273 (22): 14053-8). Receptor-ligand biosensors are also described in Hoffman et al., 2000, Proc Natl Acad Sci USA Oct 10; 97 (21): 11215-20. Optical and other biosensors comprising BACH may also be used to detect the level or presence of interaction with G- proteins and other proteins, as described by, for example, Figler et al, 1997, Biochemistry Dec 23; 36 (51): 16288-99 and Sarrio et al., 2000, Mol Cell Biol 2000 Jul ; 20 (14): 5164-74).
Sensor units for biosensors are described in, for example, US 5,492,840.

### SCREENING ASSAYS

In one embodiment of the present invention, the BACH GPCR polypeptide including functional equivalents thereof, whether natural or recombinant, is employed in a screening process for compounds which bind the receptor and which activate (agonists) or inhibit activation of (antagonists) of BACH. Thus, polypeptides used in the methods of the invention may also be used to assess the binding of small molecule substrates and ligands in, for example, cells, cell- free preparations, chemical libraries, and natural product mixtures. These substrates and ligands may be natural substrates and ligands or may be structural or functional mimetics.
See Coligan et al., Current Protocols in Immunology 1 (2): Chapter 5 (1991).

BACH GPCR polypeptides are responsible for many biological functions, including many pathologies. Accordingly, it is desirous to find compounds and drugs which stimulate BACH GPCR on the one hand and which can inhibit the function of BACH GPCR on the other hand. In general, agonists and antagonists are employed for therapeutic and prophylactic purposes for such conditions as pain and disorders of urogenital function.

Rational design of candidate compounds likely to be able to interact with BACH GPCR protein may be based upon structural studies of the molecular shapes of a polypeptide according to the invention. One means for determining which sites interact with specific other proteins is a physical structure determination, e. g., X-ray crystallography or two-dimensional NMR techniques. These will provide guidance as to which amino acid residues form molecular contact regions. For a detailed description of protein structural determination, see, e. g., Blundell and Johnson (1976) Protein Crystallography, Academic Press, New York.

An alternative to rational design uses a screening procedure which involves in general producing appropriate cells which express the BACH receptor polypeptide of the present invention on the surface thereof. Such cells include cells from animals, yeast, *Drosophila* or *E. coli.* Cells expressing the receptor (or cell membrane containing the expressed receptor) are then contacted with a test compound to observe binding, or stimulation or inhibition of a functional response. For example, *Xenopus* oocytes may be injected with BACH mRNA or polypeptide, and currents induced by exposure to test compounds measured by use of voltage clamps measured, as described in further detail elsewhere.

Furthermore, microphysiometric assays may be employed to assay BACH receptor activity. Activation of a wide variety of secondary messenger systems results in extrusion of small amounts of acid from a cell. The acid formed is largely as a result of the increased metabolic activity required to fuel the intracellular signalling process. The pH changes in the media surrounding the cell are very small but are detectable by, for example, the CYTOSENSOR microphysiometer (Molecular Devices Ltd., Menlo Park, Calif.). The CYTOSENSOR is thus capable of detecting the activation of a receptor which is coupled to an energy utilizing intracellular signaling pathway such as the G-protein coupled receptor of the present invention.

Instead of testing each candidate compound individually with the BACH receptor, a library or bank of candidate ligands may advantageously be produced and screened. Thus, for example, a bank of over 200 putative receptor ligands has been assembled for screening. The bank comprises: transmitters, hormones and chemokines known to act via a human seven transmembrane (7TM) receptor; naturally occurring compounds which may be putative agonists for a human 7TM receptor, non-mammalian, biologically active peptides for which a mammalian counterpart has not yet been identified; and compounds not found in nature, but which activate 7TM receptors with unknown natural ligands. This bank is used to screen the receptor for known ligands, using both functional (i.e. calcium, cAMP, microphysiometer, oocyte electrophysiology, etc, see elsewhere) as well as binding assays as described in further detail elsewhere. However, a large number of mammalian receptors exist for which there remains, as yet, no cognate activating ligand (agonist) or deactivating ligand (antagonist). Thus, active ligands for these receptors may not be included within the ligands banks as identified to date. Accordingly, the BACH receptor of the invention is also functionally screened (using calcium, cAMP, microphysiometer, ooyte electrophysiology, etc., functional screens) against tissue extracts to identify natural ligands. Extracts that produce positive functional responses can be sequentially subfractionated, with the fractions being assayed as described here, until an activating ligand is isolated and identified.

7TM receptors which are expressed in HEK 293 cells have been shown to be coupled functionally to activation of PLC and calcium mobilization and/or cAMP stimuation or inhibition. One screening technique therefore includes the use of cells which express the BACH GPCR receptor of this invention (for example, transfected *Xenopus* oocytes, CHO or HEK293 cells) in a system which measures extracellular pH or intracellular calcium changes caused by receptor activation. In this technique, compounds may be contacted with cells expressing the receptor polypeptide of the present invention. A second messenger response, e.g., signal transduction, pH changes, or changes in calcium level, is then measured to determine whether the potential compound activates or inhibits the receptor.

In such experiments, basal calcium levels in the HEK 293 cells in receptor-transfected or vector control cells are observed to be in the normal, 100 nM to 200 nM, range. HEK 293 cells expressing BACH GPCR or recombinant BACH GPCR are loaded with fura 2 and in a single day more than 150 selected ligands or tissue/cell extracts are evaluated for agonist induced calcium mobilization. Similarly, HEK 293 cells expressing BACH GPCR or recombinant BACH GPCR are evaluated for the stimulation or inhibition of cAMP production using standard cAMP quantitation assays. Agonists presenting a calcium transient or cAMP fluctuation are tested in vector control cells to determine if the response is unique to the transfected cells expressing receptor.

Another method involves screening for receptor inhibitors by determining inhibition or stimulation of BACH receptor-mediated cAMP and/or adenylate cyclase accumulation. Such a method involves transfecting a eukaryotic cell with the receptor of this invention to express the receptor on the cell surface. The cell is then exposed to potential antagonists in the presence of the receptor of this invention. The amount of cAMP accumulation is then measured. If the potential antagonist binds the receptor, and thus inhibits receptor binding, the levels of receptor-mediated cAMP, or adenylate cyclase, activity will be reduced or increased.

Another method for detecting agonists or antagonists for the receptor of the present invention is the yeast based technology as described in U. S. Pat. No. 5,482,835.

Where the candidate compounds are proteins, in particular antibodies or peptides, libraries of candidate compounds may be screened using phage display techniques. Phage display is a protocol of molecular screening which utilises recombinant bacteriophage. The technology involves transforming bacteriophage with a gene that encodes one compound from the library of candidate compounds, such that each phage or phagemid expresses a particular candidate compound. The transformed bacteriophage (which preferably is tethered to a solid support) expresses the appropriate candidate compound and displays it on their phage coat. Specific candidate compounds which are capable of binding to a polypeptide or peptide of the invention are enriched by selection strategies based on affinity interaction. The successful candidate agents are then characterised. Phage display has advantages over standard affinity ligand screening technologies. The phage surface displays the candidate agent in a three dimensional configuration, more closely resembling its naturally occurring conformation. This allows for more specific and higher affinity binding for screening purposes.

Another method of screening a library of compounds utilises eukaryotic or prokaryotic host cells which are stably transformed with recombinant DNA molecules expressing a library of compounds. Such cells, either in viable or fixed form, can be used for standard binding-partner assays. See also Parce et al. (1989) Science 246:243-247; and Owicki et al. (1990) Proc. Nat'l Acad. Sci. USA 87;4007-4011, which describe sensitive methods to detect cellular responses. Competitive assays are particularly useful, where the cells expressing the library of compounds are contacted or incubated with a labelled antibody known to bind to a BACH polypeptide of the present invention, such as ¹²⁵I-antibody, and a test sample such as a candidate compound whose binding affinity to the binding composition is being measured. The bound and free labelled binding partners for the polypeptide are then separated to assess the degree of binding. The amount of test sample bound is inversely proportional to the amount of labelled antibody binding to the polypeptide.

Any one of numerous techniques can be used to separate bound from free binding partners to assess the degree of binding. This separation step could typically involve a procedure such as adhesion to filters followed by washing, adhesion to plastic following by washing, or centrifugation of the cell membranes.

Still another approach is to use solubilized, unpurified or solubilized purified polypeptide or peptides, for example extracted from transformed eukaryotic or prokaryotic host cells. This allows for a "molecular" binding assay with the advantages of increased specificity, the ability to automate, and high drug test throughput.

Another technique for candidate compound screening involves an approach which provides high throughput screening for new compounds having suitable binding affinity, e.g., to a polypeptide of the invention, and is described in detail in International Patent application no. WO 84/03564 (Commonwealth Serum Labs.), published on September 13 1984. First, large numbers of different small peptide test compounds are synthesized on a solid substrate, e.g., plastic pins or some other appropriate surface; see Fodor *et al.* (1991). Then all the pins are reacted with solubilized polypeptide of the invention and washed. The next step involves detecting bound polypeptide. Compounds which interact specifically with the polypeptide will thus be identified.

Ligand binding assays provide a direct method for ascertaining receptor pharmacology and are adaptable to a high throughput format. The purified ligand for a receptor may be radiolabeled to high specific activity (50-2000 Ci/mmol) for binding studies. A determination is then made that the process of radiolabeling does not diminish the activity of the ligand towards its receptor. Assay conditions for buffers, ions, pH and other modulators such as nucleotides are optimized to establish a workable signal to noise ratio for both membrane and whole cell receptor sources. For these assays, specific receptor binding is defined as total associated radioactivity minus the radioactivity measured in the presence of an excess of unlabeled competing ligand. Where possible, more than one competing ligand is used to define residual nonspecific binding.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed.

Further, the assays may simply comprise the steps of mixing a candidate compound with a solution containing a BACH GPCR polypeptide to form a mixture, measuring BACH GPCR activity in the mixture, and comparing the BACH GPCR activity of the mixture to a standard.

The BACH GPCR cDNA, protein and antibodies to the protein may also be used to configure assays for detecting the effect of added compounds on the production of BACH GPCR mRNA and protein in cells. For example, an ELISA may be constructed for measuring secreted or cell associated levels of BACH GPCR protein using monoclonal and polyclonal antibodies by standard methods known in the art, and this can be used to discover agents which may inhibit or enhance the production of BACH GPCR (also called antagonist or agonist, respectively) from suitably manipulated cells or tissues. Standard methods for conducting screening assays are well understood in the art.

Examples of potential BACH GPCR antagonists include antibodies or, in some cases, nucleotides and their analogues, including purines and purine analogues, oligonucleotides or proteins which are closely related to the ligand of the BACH GPCR, e. g., a fragment of the ligand, or small molecules which bind to the receptor but do not elicit a response, so that the activity of the receptor is prevented.

Preferably, the agonists or antagonist comprises an antibody capable of binding specifically to a BACH polypeptide having an amino acid sequence shown in SEQ ID NO: 3, SEQ ID NO: 5, SEQ ID NO: 7 or SEQ ID NO: 9 or a functionally equivalent sequence having at least 90% sequence identity thereto.

The term"compound"refers to a chemical compound (naturally occurring or synthesised), such as a biological macromolecule (e. g., nucleic acid, protein, non-peptide, or organic molecule), or an extract made from biological materials such as bacteria, plants, fungi, or animal (particularly mammalian) cells or tissues, or even an inorganic element or molecule. Preferably the compound is an antibody.

### TRANSGENIC ANIMALS

The present invention further encompasses use of a non-human animal which displays a decrease in sensitivity to pain or a hypoactive bladder when compared to a wild-type animal, the non-human animal being a transgenic animal having a functionally disrupted endogenous BACH gene, wherein the BACH gene comprises a nucleic acid sequence shown in SEQ ID NO: 1, SEQ ID NO: 2, SEQ ID NO: 4, SEQ ID NO: 10 or a functionally equivalent sequence having at least 90% sequences identity thereto, to identify an agonist or antagonist of the BACH polypeptide for the treatment or alleviation of pain or a disorder of urogenital function. Included are transgenic animals ("BACH knockout"s) which do not express functional BACH receptor.
The BACH knockouts may arise as a result of functional disruption of the BACH gene or any portion of that gene, including one or more loss of function mutations, including a deletion or replacement, of the BACH gene. The mutations include single point mutations, and may target coding or non-coding regions of BACH.

Preferably, such a non-human mammal is a mouse. Such non-human animals may be used as a model for pain or a disorder of urogenital function.

Mice which are null for BACH may be used for various purposes. For example, transgenic animals that have been engineered to be deficient in the production of BACH GPCR may be used in assays to identify agonists and/or antagonists of BACH GPCR. One assay is designed to evaluate a potential drug (aa candidate ligand or compound) to determine if it produces a physiological response in the absence of BACH GPCR receptors. This may be accomplished by administering the drug to a transgenic animal as discussed above, and then assaying the animal for a particular response. Although any physiological parameter could be measured in this assay, preferred responses include one or more of the following: changes to disease resistance; altered inflammatory responses; altered tumour susceptability: a change in blood pressure; neovascularization; a change in eating behavior; a change in body weight; a change in bone density; a change in body temperature; insulin secretion; gonadotropin secretion; nasal and bronchial secretion; vasoconstriction; loss of memory; anxiety; hyporeflexia or hyperreflexia; pain or stress responses.

Tissues derived from the BACH knockout animals may be used in receptor binding assays to determine whether the potential drug (a candidate ligand or compound) binds to the BACH receptor. Such assays can be conducted by obtaining a first receptor preparation from the transgenic animal engineered to be deficient in BACH receptor production and a second receptor preparation from a source known to bind any identified BACH ligands or compounds. In general, the first and second receptor preparations will be similar in all respects except for the source from which they are obtained. For example, if brain tissue from a transgenic animal (such as described above and below) is used in an assay, comparable brain tissue from a normal (wild type) animal is used as the source of the second receptor preparation. Each of the receptor preparations is incubated with a ligand known to bind to BACH receptors, both alone and in the presence of the candidate ligand or compound. Preferably, the candidate ligand or compound will be examined at several different concentrations.

The extent to which binding by the known ligand is displaced by the test compound is determined for both the first and second receptor preparations. Tissues derived from transgenic animals may be used in assays directly or the tissues may be processed to isolate membranes or membrane proteins, which are themselves used in the assays. A preferred transgenic animal is the mouse. The ligand may be labeled using any means compatible with binding assays. This would include, without limitation, radioactive, enzymatic, fluorescent or chemiluminescent labeling (as well as other labelling techniques as described in further detail above).

Furthermore, antagonists of BACH GPCR receptor may be identified by administering candidate compounds, etc, to wild type animals expressing functional BACH, and animals identified which exhibit any of the phenotypic characteristics associated with reduced or abolished expression of BACH receptor function.

Detailed methods for generating non-human transgenic animal are described in further detail below. Transgenic gene constructs can be introduced into the germ line of an animal to make a transgenic mammal. For example, one or several copies of the construct may be incorporated into the genome of a mammalian embryo by standard transgenic techniques.

In an exemplary embodiment, the transgenic non-human animals of the invention are produced by introducing transgenes into the germline of the non-human animal. Embryonal target cells at various developmental stages can be used to introduce transgenes. Different methods are used depending on the stage of development of the embryonal target cell. The specific line(s) of any animal used to practice this invention are selected for general good health, good embryo yields, good pronuclear visibility in the embryo, and good reproductive fitness. In addition, the haplotype is a significant factor.

Introduction of the transgene into the embryo can be accomplished by any means known in the art such as, for example, microinjection, electroporation, or lipofection. For example, the BACH receptor transgene can be introduced into a mammal by microinjection of the construct into the pronuclei of the fertilized mammalian egg(s) to cause one or more copies of the construct to be retained in the cells of the developing mammal(s). Following introduction of the transgene construct into the fertilized egg, the egg may be incubated in vitro for varying amounts of time, or reimplanted into the surrogate host, or both. *In vitro* incubation to maturity is within the scope of this invention. One common method in to incubate the embryos in vitro for about 1-7 days, depending on the species, and then reimplant them into the surrogate host.

The progeny of the transgenically manipulated embryos can be tested for the presence of the construct by Southern blot analysis of the segment of tissue. If one or more copies of the exogenous cloned construct remains stably integrated into the genome of such transgenic embryos, it is possible to establish permanent transgenic mammal lines carrying the transgenically added construct.

The litters of transgenically altered mammals can be assayed after birth for the incorporation of the construct into the genome of the offspring. Preferably, this assay is accomplished by hybridizing a probe corresponding to the DNA sequence coding for the desired recombinant protein product or a segment thereof onto chromosomal material from the progeny. Those mammalian progeny found to contain at least one copy of the construct in their genome are grown to maturity.

For the purposes of this invention a zygote is essentially the formation of a diploid cell which is capable of developing into a complete organism. Generally, the zygote will be comprised of an egg containing a nucleus formed, either naturally or artificially, by the fusion of two haploid nuclei from a gamete or gametes. Thus, the gamete nuclei must be ones which are naturally compatible, i.e., ones which result in a viable zygote capable of undergoing differentiation and developing into a functioning organism. Generally, a euploid zygote is preferred. If an aneuploid zygote is obtained, then the number of chromosomes should not vary by more than one with respect to the euploid number of the organism from which either gamete originated.

In addition to similar biological considerations, physical ones also govern the amount (e.g., volume) of exogenous genetic material which can be added to the nucleus of the zygote or to the genetic material which forms a part of the zygote nucleus. If no genetic material is removed, then the amount of exogenous genetic material which can be added is limited by the amount which will be absorbed without being physically disruptive. Generally, the volume of exogenous genetic material inserted will not exceed about 10 picoliters. The physical effects of addition must not be so great as to physically destroy the viability of the zygote. The biological limit of the number and variety of DNA sequences will vary depending upon the particular zygote and functions of the exogenous genetic material and will be readily apparent to one skilled in the art, because the genetic material, including the exogenous genetic material, of the resulting zygote must be biologically capable of initiating and maintaining the differentiation and development of the zygote into a functional organism.

The number of copies of the transgene constructs which are added to the zygote is dependent upon the total amount of exogenous genetic material added and will be the amount which enables the genetic transformation to occur. Theoretically only one copy is required; however, generally, numerous copies are utilized, for example, 1,000-20,000 copies of the transgene construct, in order to insure that one copy is functional. As regards the present invention, there will often be an advantage to having more than one functioning copy of each of the inserted exogenous DNA sequences to enhance the phenotypic expression of the exogenous DNA sequences.

Any technique which allows for the addition of the exogenous genetic material into nucleic genetic material can be utilized so long as it is not destructive to the cell, nuclear membrane or other existing cellular or genetic structures. The exogenous genetic material is preferentially inserted into the nucleic genetic material by microinjection. Microinjection of cells and cellular structures is known and is used in the art.

Reimplantation is accomplished using standard methods. Usually, the surrogate host is anesthetized, and the embryos are inserted into the oviduct. The number of embryos implanted into a particular host will vary by species, but will usually be comparable to the number of off spring the species naturally produces.

Transgenic offspring of the surrogate host may be screened for the presence and/or expression of the transgene by any suitable method. Screening is often accomplished by Southern blot or Northern blot analysis, using a probe that is complementary to at least a portion of the transgene. Western blot analysis using an antibody against the protein encoded by the transgene may be employed as an alternative or additional method for screening for the presence of the transgene product. Typically, DNA is prepared from tail tissue and analyzed by Southern analysis or PCR for the transgene. Alternatively, the tissues or cells believed to express the transgene at the highest levels are tested for the presence and expression of the transgene using Southern analysis or PCR, although any tissues or cell types may be used for this analysis.

Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

Progeny of the transgenic animals may be obtained by mating the transgenic animal with a suitable partner, or by in vitro fertilization of eggs and/or sperm obtained from the transgenic animal. Where mating with a partner is to be performed, the partner may or may not be transgenic and/or a knockout; where it is transgenic, it may contain the same or a different transgene, or both. Alternatively, the partner may be a parental line. Where in vitro fertilization is used, the fertilized embryo may be implanted into a surrogate host or incubated in vitro, or both. Using either method, the progeny may be evaluated for the presence of the transgene using methods described above, or other appropriate methods.

The transgenic animals produced in accordance with the present invention will include exogenous genetic material. As set out above, the exogenous genetic material will, in certain embodiments, be a DNA sequence which results in the production of a BACH GPCR receptor. Further, in such embodiments the sequence will be attached to a transcriptional control element, e.g., a promoter, which preferably allows the expression of the transgene product in a specific type of cell.

Retroviral infection can also be used to introduce transgene into a non-human animal. The developing non-human embryo can be cultured in vitro to the blastocyst stage. During this time, the blastomeres can be targets for retroviral infection (Jaenich, R. (1976) PNAS 73:1260-1264). Efficient infection of the blastomeres is obtained by enzymatic treatment to remove the zona pellucida (Manipulating the Mouse Embryo, Hogan eds. (Cold Spring Harbor Laboratory Press, Cold Spring Harbor, 1986). The viral vector system used to introduce the transgene is typically a replication-defective retrovirus carrying the transgene (Jahner et al. (1985) PNAS 82:6927-6931; Van der Putten et al. (1985) PNAS 82:6148-6152). Transfection is easily and efficiently obtained by culturing the blastomeres on a monolayer of virus-producing cells (Van der Putten, supra; Stewart et al. (1987) EMBO J. 6:383-388). Alternatively, infection can be performed at a later stage. Virus or virus-producing cells can be injected into the blastocoele (Jahner et al. (1982) Nature 298:623-628). Most of the founders will be mosaic for the transgene since incorporation occurs only in a subset of the cells which formed the transgenic non-human animal. Further, the founder may contain various retroviral insertions of the transgene at different positions in the genome which generally will segregate in the offspring. In addition, it is also possible to introduce transgenes into the germ line by intrauterine retroviral infection of the midgestation embryo (Jahner et al. (1982) supra).

A third type of target cell for transgene introduction is the embryonal stem cell (ES). ES cells are obtained from pre-implantation embryos cultured in vitro and fused with embryos (Evans et al. (1981) Nature 292: 154-156; Bradley et al. (1984) Nature 309: 255-258; Gossler et al. (1986) PNAS 83 : 9065-9069; and Robertson et al. (1986) Nature 322: 445-448). Transgenes can be efficiently introduced into the ES cells by DNA transfection or by retrovirus-mediated transduction. Such transformed ES cells can thereafter be combined with blastocysts from a non-human animal. The ES cells thereafter colonize the embryo and contribute to the germ line of the resulting chimeric animal. For review see Jaenisch, R. (1988) Science 240: 1468-1474. BACH GPCR knock-outs may be generated by various means known in the art, as described in further detail below. A specific description of the construction of a BACH knock-out mouse is disclosed in Example 2 below.

A nucleic acid construct for functionally disrupting a BACH gene in a host cell may comprises: a) a non-homologous replacement portion; b) a first homology region located upstream of the non-homologous replacement portion, the first homology region having a nucleotide sequence with substantial identity to a first BACH gene sequence; and c) a second homology region located downstream of the non-homologous replacement portion, the second homology region having a nucleotide sequence with substantial identity to a second BACH gene sequence, the second BACH gene sequence having a location downstream of the first BACH gene sequence in a naturally occurring endogenous BACH gene. Additionally, the first and second homology regions are of sufficient length for homologous recombination between the nucleic acid construct and an endogenous BACH gene in a host cell when the nucleic acid molecule is introduced into the host cell. Preferably, the non-homologous replacement portion comprises an expression reporter, preferably including lacZ and a positive selection expression cassette, preferably including a neomycin phosphotransferase gene operatively linked to a regulatory element (s).

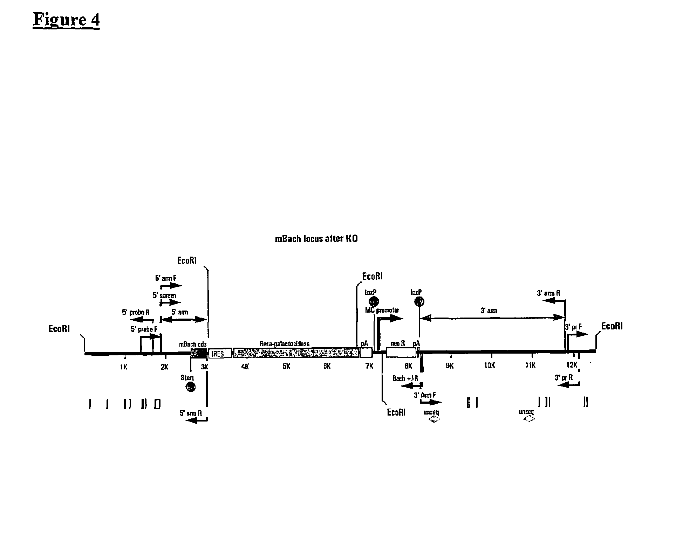

Preferably, the first and second BACH gene sequences are derived from SEQ ID No. 1, SEQ ID No. 2 or SEQ ID NO: 4, or a mouse BACH genomic sequence (SEQ ID NO: 10), or a functionally equivalent sequence having at least 90% sequence identity thereto. Preferably, the construct comprises a structure depicted in Figure 4.

The host cell can be a mammalian cell that normally expresses BACH from the liver, brain, spleen or heart, or a pluripotent cell, such as a mouse embryonic stem cell. Further development of an embryonic stem cell into which the nucleic acid construct has been introduced and homologously recombined with the endogenous BACH gene produces a transgenic nonhuman animal having cells that are descendant from the embryonic stem cell and thus carry the BACH gene disruption in their genome. Animals that carry the BACH gene disruption in their germline can then be selected and bred to produce animals having the BACH gene disruption in all somatic and germ cells. Such mice can then be bred to homozygosity for the BACH gene disruption.

A BACH GPCR deficient transgenic animal may be generated as follows:

### Construction of BACH Gene Targeting Vector

Murine BACH genomic clones are isolated from a mouse large insert PAC library obtained from HGMP (Hinxton, UK) using the human open reading frame cDNA sequence (SEQ ID NO: 1) as a probe using standard techniques. The isolated murine BACH genomic clones are then restriction mapped in the region of the BACH gene using small oligonucleotide probes and standard techniques. A mouse genomic BACH sequence is depicted as SEQ ID NO: 10.

The murine genomic locus is partially sequenced to enable the design of homologous arms to clone into the targeting vector. The murine BACH gene is a single exon gene. A 5' homologous arm and a 3' homologous arm are amplified by PCR and the fragment cloned into the targeting vector. Any suitable size may be chosen for the length of these arms to enable homologous recombination; for example, the 5' arm may be between 1 kb and to 2 kb, for example 1.15 kb, while the 3' arm may be about 4 kb in size.

The position of these arms is chosen to functionally disrupt the BACH gene by deleting the seven transmembrane spanning regions. A targeting vector is prepared where the deleted BACH sequence is replaced with non-homologous sequences composed of an endogenous gene expression reporter (an in frame fusion with lacZ) upstream of a selection cassette composed of a self promoted neomycin phosphotransferase (neo) gene in the same orientation as the BACH gene.

### Transfection and Analysis of Embryonal Stem Cells

Embryonal stem cells (Evans and Kaufman, 1981) are cultured on a neomycin resistant embryonal fibroblast feeder layer grown in Dulbecco's Modified Eagles medium supplemented with 20% Fetal Calf Serum, 10% new-born calf serum, 2 mM glutamine, non-essential amino acids, 100 LM 2-mercaptoethanol and 500 u/ml leukemia inhibitory factor. Medium is changed daily and ES cells are subcultured every three days. 5x10 ES cells are transfected with 5 pg of linearized plasmid by electroporation (25 I1F capacitance and 400 Volts). 24 hours following electroporation the transfected cells are cultured for 9 days in medium containing 200 j. g/ml neomycin. Clones are picked into 96 well plates, replicated and expanded before being screened by PCR to identify clones in which homologous recombination had occurred between the endogenous BACH gene and the targeting construct. From 200 picked clones 7 targets are identified. These clones where expanded to allow replicas to be frozen and sufficient high quality DNA to be prepared for Southern blot confirmation of the targeting event using external 5'and 3' probes, all using standard procedures (Russ et al, 2000)

### Generation of BACH GPCR Defzcient Mice

C57BL/6 female and male mice are mated and blastocysts are isolated at 3.5 days of gestation. 10-12 cells from a chosen clone are injected per blastocyst and 7-8 blastocysts are implanted in the uterus of a pseudopregnant F1 female. Five chimeric pups are born of which one male is 100% agouti (indicating cells descendent from the targeted clone). This male chimera is mated with female and MF1 and 129 mice, and germline transmission is determined by the agouti coat color and by PCR genotyping respectively.

### ANTIBODIES

The present invention relates to antibodies which bind to a BACH polypeptide.

For the purposes of this invention, the term "antibody", unless specified to the contrary, includes but is not limited to, polyclonal, monoclonal, chimeric, single chain, Fab fragments and fragments produced by a Fab expression library. Such fragments include fragments of whole antibodies which retain their binding activity for a target substance, Fv. F (ab') and F (ab') 2 fragments, as well as single chain antibodies (scFv), fusion proteins and other synthetic proteins which comprise the antigen-binding site of the antibody. The antibodies and fragments thereof may be humanised antibodies, for example as described in EP-A-239400. Furthermore, antibodies with fully human variable regions (or their fragments), for example, as described in US Patent Nos. 5,545,807 and 6,075,181 may also be used. Neutralizing antibodies, i. e., those which inhibit any biological activity of BACH. are especially preferred for diagnostics and therapeutics.

Antibodies may be produced by standard techniques, such as by immunisation or by using a phage display library.

### DIAGNOSTIC ASSAYS

This invention also relates to the use of BACH GPCR polynucleotides and polypeptides (as well as functional equivalents having at least 90% sequence identity thereto) for use in diagnosis as diagnostic reagents or in genetic analysis.

We provide for a natural variant of BACH polypeptide or nucleic acid, and the use of such a natural variant in diagnosis of BACH associated disease. BACH polymorphisms may include differences at the nucleic acid level, which may or may not reflect differences in the amino acid level. Preferably, such BACH variants or mutants are such that they include changes in the amino acid level. However, the invention also encompasses BACH polymorphisms which occur in non-coding regions, for example, expression control regions such as promoters and enhancers.

Polymorphisms in BACH include deletions of one or more nucleic acids, insertions of one or more nucleic acids, inversions, etc. Preferably, BACH polymorphisms comprise single nucleotide polymorphisms.

Polymorphisms in BACH may be identified by comparing sequences at the appropriate level (whether nucleic acid or protein) between individuals in a population. Differences in sequences may be reflected in different physical properties, and techniques for detecting these may rely on detection of changes in physical properties. For example, single nucleotide polymorphisms may be detected as restriction fragment length polymorphisms (i.e., difference in susceptibility to digestion by a restriction enzyme). Furthermore, SNPS may affect the migration or mobility of a nucleic acid fragment or protein fragment in a gel.

Non-coding polymorphisms in BACH may be identified by sequencing non-coding regions of BACH. For example, control regions of the BACH gene, such as enhancers and promoters may be sequenced to identify polymorphisms. The effect of such non-coding polymorphisms on the expression level of BACH may be determined by constructing transgenic mice (as described below) comprising the mutant BACH sequences, or by generating expression constructs and transfection into cell lines. In each case, the expression level of BACH is detected, by RT-PCR or antibody Western staining, to determine the effect of the mutation in the control of expression of BACH. Useful BACH polymorphisms are those which modulate the level of expression, wiether by up-regulation or down-regulation of BACH levels.

Accordingly, this invention provides for a variant or mutant or polymorphism in a non-coding region of BACH, preferably in a control region of BACH, preferably in a promoter and/or enhancer of BACH, which is capable of modulating the level of expression of BACH in an organism. The invention also provides for a set of two or more of such mutants or variants or polymorphisms, preferably non-coding polymorphisms. The invention also provides for the use of such variants or polymorphisms or sets of variants to identify nucleic acid and/or amino acid positions, in which changes to such positions affect the level of expression of BACH. The invention also provides for a transgenic animal comprising a variant or mutant or polymorphism of BACH, preferably, a non-coding polymorphism.

Detection of a mutated form of the BACH GPCR gene associated with a dysfunction will provide a diagnostic tool that can add to or define a diagnosis of a disease or susceptibility to a disease which results from under-expression, over-expression or altered expression of BACH GPCR. Individuals carrying mutations in the BACH GPCR gene (including control sequences) may be detected at the DNA level by a variety of techniques.

For example, DNA may be isolated from a patient and the DNA polymorphism pattern of BACH determined. The identified pattern is compared to controls of patients known to be suffering from a disease associated with over-, under-or abnormal expression of BACH. Patients expressing a genetic polymorphism pattern associated with BACH associated disease may then be identified. Genetic analysis of the BACH GPCR gene may be conducted by any technique known in the art. For example, individuals may be screened by determining DNA sequence of a BACH allele, by RFLP or SNP analysis, etc.
Patients may be identified as having a genetic predisposition for a disease associated with the over-, under-, or abnormal expression of BACH by detecting the presence of a DNA polymorphism in the gene sequence for BACH or any sequence controlling its expression.

Patients so identified can then be treated to prevent the occurrence of BACH associated disease, or more aggressively in the early stages of BACH associated disease to prevent the further occurrence or development of the disease. BACH associated diseases include pain and disorders of urogenital function.

Nucleic acids for diagnosis may be obtained from a subject's cells, such as from blood, urine, saliva, tissue biopsy or autopsy material. In a preferred embodiment, the DNA is obtained from blood cells obtained from a finger prick of the patient with the blood collected on absorbent paper. In a further preferred embodiment, the blood is collected on an AmpliCard. TM. (University of Sheffield, Department of Medicine and Pharmacology, Royal Hallamshire Hospital, Sheffield, England S 10 2JF).

The DNA may be used directly for detection or may be amplified enzymatically by using PCR or other amplification techniques prior to analysis. Oligonucleotide DNA primers that target the specific polymorphic DNA region within the genes of interest may be prepared so that in the PCR reaction amplification of the target sequences is achieved.
RNA or cDNA may also be used as templates in similar fashion. The amplified DNA sequences from the template DNA may then be analyzed using restriction enzymes to determine the genetic polymorphisms present in the amplified sequences and thereby provide a genetic polymorphism profile of the patient. Restriction fragments lengths may be identified by gel analysis. Alternatively, or in conjunction, techniques such as. SNP (single nucleotide polymorphisms) analysis may be employed.

Deletions and insertions can be detected by a change in size of the amplified product in comparison to the normal genotype. Point mutations can be identified by hybridizing amplified DNA to labeled BACH GPCR nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, eg., Myers et al, Science (1985) 230: 1242. Sequence changes at specific locations may also be revealed by nuclease protection assays, such as RNase and Slprotection or the chemical cleavage method. See Cotton et al., Proc Natl Acad Sci USA (1985) 85 : 4397-4401. In another embodiment, an array of oligonucleotides probes comprising the BACH GPCR nucleotide sequence or fragments thereof can be constructed to conduct efficient screening of e. g., genetic mutations. Array technology methods are well known and have general applicability and can be used to address a variety of questions in molecular genetics including gene expression, genetic linkage, and genetic variability. (See for example: M. Chee et al., Science, Vol 274, pp 610-613 (1996)).

Single strand conformation polymorphism (SSCP) may be used to detect differences in electrophoretic mobility between mutant and wild type nucleic acids (Orita et al. (1989) Proc Natl. Acad. Sci USA: 86: 2766, see also Cotton (1993) Mutat Res 285: 125-144; and Hayashi (1992) Genet Anal Tech Appl 9: 73-79). Single-stranded DNA fragments of sample and control BACH nucleic acids may be denatured and allowed to nature. The secondary structure of single-stranded nucleic acids varies according to sequence, the resulting alteration in electrophoretic mobility enables the detection of even a single base change. The DNA fragments may be labeled or detected with labeled probes.
The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7: 5).

The diagnostic assays offer a process for diagnosing or determining a susceptibility to BACH associated diseases, for example, pain and disorders of urogenital function.

The presence of BACH GPCR polypeptides and nucleic acids may be detected in a sample. Thus, infections and diseases as listed above can be diagnosed by methods comprising determining from a sample derived from a subject an abnormally decreased or increased level of the BACH GPCR polypeptide or BACH GPCR mRNA. The sample may comprise a cell or tissue sample from an organism suffering or suspected to be suffering from a disease associated with increased, reduced or otherwise abnormal BACH GPCR expression, including spatial or temporal changes in level or pattern of expression.
The level or pattern of expression of BACH in an organism suffering from or suspected to be suffering from such a disease may be usefully compared with the level or pattern of expression in a normal organism as a means of diagnosis of disease.

In general therefore, the invention includes a method of detecting polymorphism of a nucleic acid comprising a BACH GPCR nucleic acid in a sample, by contacting the sample with at least one nucleic acid probe which is specific for said nucleic acid and monitoring said sample for the presence of polymorphism of the nucleic acid. For example, the nucleic acid probe may specifically bind to the BACH GPCR nucleic acid, or a portion of it, and binding between the two detected; the presence of the complex itself may also be detected.
Furthermore, the invention encompasses a method of detecting the presence of a BACH GPCR polypeptide by contacting a cell sample with an antibody capable of binding the polypeptide and monitoring said sample for the presence of the polypeptide. This may conveniently be achieved by monitoring the presence of a complex formed between the antibody and the polypeptide, or monitoring the binding between the polypeptide and the antibody. Methods of detecting binding between two entities are known in the art, and include FRET (fluorescence resonance energy transfer), surface plasmon resonance, etc.

Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as a BACH GPCR, in a sample derived from a host are well-known to those of skill in the art.
Such assay methods include radioimmunoassays, competitive-binding assays, Western Blot analysis and ELISA assays.

### EXAMPLES

### Example 1. Transgenic BACH Knock-Out Mouse

### Construction of BACH Gene Targeting Vector

Murine BACH genomic clones are isolated from a mouse large insert PAC library obtained from HGMP (Hinxton, UK) using the human open reading frame cDNA sequence (SEQ ID NO: 1) as a probe using standard techniques.

A genomic sequence of mouse BACH GPCR is shown in SEQ ID NO: 10.

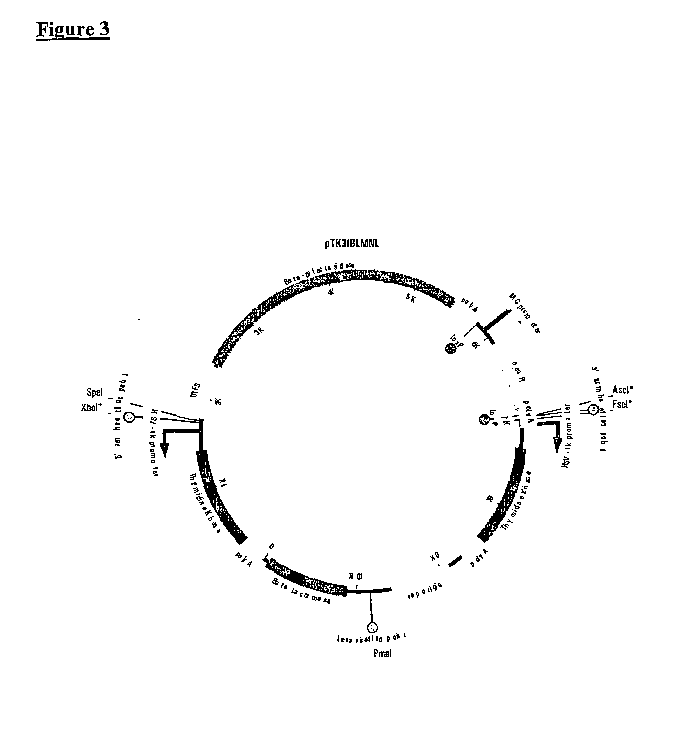

The isolated murine BACH genomic clones are then restriction mapped in the region of the BACH gene using small oligonucleotide probes and standard techniques. The murine genomic locus is partially sequenced to enable the design of homologous arms to clone into the targeting vector. The murine BACH gene is a single exon gene. A 1.15 kb 5'homologous arm and an approximately 4 kb 3'homologous arm are amplified by PCR and the fragments are cloned into the targeting vector. Each of the primers used to amplify the arms is synthesized to contain the recognition site for a rare-cutting restriction enzyme, positioned at the 5'end of each oligonucleotide. The incorporation of restriction sites into the arms during PCR amplification facilitates cloning of the resultant fragments into the standard targeting vector, as the vector contains mutually compatible unique sites for each enzyme. In the case of BACH, the primers are designed as listed in the sequence table below. The cloning enzymes used are as follows: 5'armF XhoI, 5'armR Spel, 3'armF AscI and 3'armR FseI. The fragments are cloned in to the vector known as pTK3IBLMNL depicted in Figure 3.

In addition to the arm primer pairs (5'armF/5'armR and 3'armF/3'armR), primers specific to the BACH locus are also designed for the following purposes: 5' and 3' probe primer pairs (5'prF/5'prR and 3'prF/3'prR) to amplify a short 200-300bp fragment of non-repetitive genomic DNA external to and extending beyond each arm, to allow Southern analysis of the targeted locus, in isolated putative targeted clones; a mouse genotyping primer pair (+/-F and +/-R) which allows differentiation between wild-type, heterozygote and homozygouse mice; and lastly, a target screening primer (5'scr) which anneals upstream of the beginning of the 5' arm region, and which produces a target event specific 1.2kb amplimer when paired with a primer specific to the 5' end of the vector (DR1). This amplimer can only be derived from template DNA from cells where the desired genomic alteration has occurred and allows the identification of correctly targeted cells from the background of clones containing randomly integrated copies of the vector.

**Table 1. BACH Primer Sequences**

| | |
|---|---|
| mBach 5' pr F | GAGCTGAGGATGTAATCCTAGCACTTG |
| mBach 5' pr R | CCTTCCTTACTAAACTGTGGGGCACTC |
| mBach 5' scr | GGGTCACCACAACTGTATGAAAGAGTC |
| mBach 5' arm F | AAACTCGAGAGGAAAGCTGAGAATCACTGCCTTGAG |
| mBach 5' arm R | AAAACTAGTCGATAGTCAGGGCACTGGAGCACAGAG |
| mBach 3' arm F | TTTGGCGCGCCTGAGGGAGGTGGGCTGGGTACTTGGAC |
| mBach 3' arm R | AAAGGCCGGCCACCACCTTCTGCTTTCTCTTACCTTC |
| mBach 3' pr F | AGGTTCGCAGACATGCTTGTAGGATAG |
| mBach 3' pr R | GGGGCACTTTGTGAAAATGGCAGACTC |
| mBach +/-F | GCTCACCGAACTACCCTCAGAAAGCAC |
| mBach +/-R | CCCTTCTGCACACTAGAGCTGGAGTTG |

The position of the homology arms is chosen to functionally disrupt the BACH gene by deleting the seven transmembrane spanning regions. A targeting vector is prepared where the deleted BACH sequence is replaced with non-homologous sequences composed of an endogenous gene expression reporter (a frame independent fusion with lacZ) upstream of a selection cassette composed of promoted neomycin phosphotransferase (neo) gene in the same orientation as the BACH gene.

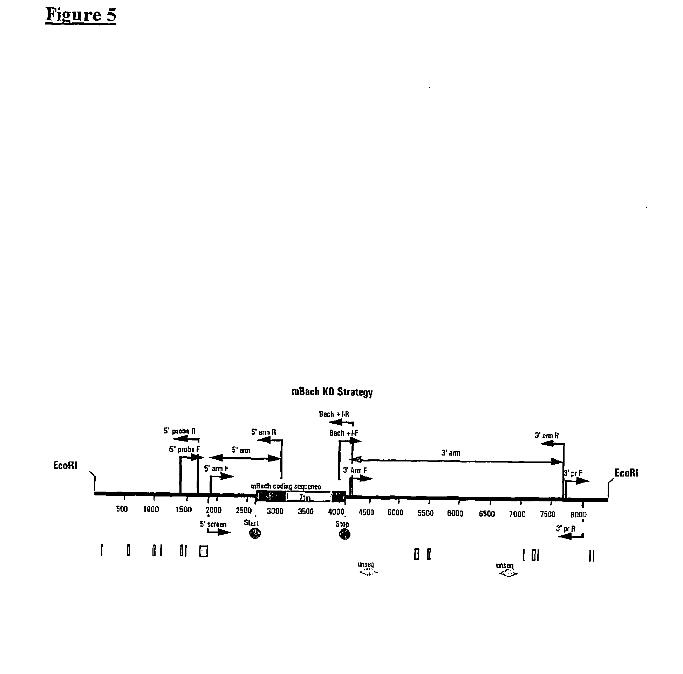

Recombination of the vector into the murine genome at the correct locus generates an allele for BACH with the structure depicted in Figure 4. For comparison, the structure of a wild-type allele for BACH is show in in Figure 5. The sequence of the entire genomic contig used in this strategy to prepare the transgenic BACH knock-out mouse is depicted in a separate section below. Regions corresponding to the primers are annotated.

It should be noted that the entire 7tm region has been removed in the knockout allele (Figure 4). The sites for the enzyme EcoRI are included as this enzyme is used to confirm the correct integration of the targeting construct using Southern analysis of putative clones.

Once the 5' and 3' homology arms are cloned into the targeting vector, a large highly pure DNA preparation is made using standard molecular biology techniques. 20 µg of the freshly prepared endotoxin free DNA is restricted with another rare-cutting restriction enzyme, PmeI, present at a unique site in the vector backbone between the amplicillin resistance gene and the bacterial origin of replication. The linearized DNA is then precipitated and resuspended in 100 µl of Phosphate Buffered Saline, ready for electroporation.

### Transfection and Analysis of Embryonal Stem Cells

Embryonal stem cells (Evans and Kaufman, 1981^{I}) are cultured on a neomycin resistant embryonal fibroblast feeder layer grown in Dulbecco's Modified Eagles medium supplemented with 20% Fetal Calf Serum, 10% new-born calf serum, 2 mM glutamine, non-essential amino acids, 100 µM 2-mercaptoethanol and 500 u/ml leukemia inhibitory factor. Medium is changed daily and ES cells are sub-cultured every three days. 5x10⁶ ES cells are transfected with 20 µg of linearized plasmid by electroporation (25 µF capacitance and 400 Volts).

24 hours following electroporation the transfected cells are cultured for 9 days in medium containing 200 µg/ml neomycin. Clones are picked into 96 well plates, replicated and expanded before being screened by PCR (using primers 5'scr and DR1, as described above) to identify clones in which homologous recombination had occurred between the endogenous BACH gene and the targeting construct. From 200 picked clones 7 targets are identified. These clones are expanded to allow replicas to be frozen and sufficient high quality DNA to be prepared for Southern blot confirmation of the targeting event using the external 5' and 3' probes prepared as described above, all using standard procedures (Russ et al, 2000)

### Generation of BACH GPCR Deficient Mice

C57BL/6 female and male mice are mated and blastocysts are isolated at 3.5 days of gestation. 10-12 cells from a chosen clone are injected per blastocyst and 7-8 blastocysts are implanted in the uterus of a pseudopregnant F1 female. Five chimeric pups are bom of which one male is 100% agouti (indicating cells descendent from the targeted clone). This male chimera is mated with female and MF1 and 129 mice, and germline transmission is determined by the agouti coat colour and by PCR genotyping respectively.

Genotyping is carried out by PCR on lysed tail clips, using the primers +/-F and +/R with a third, neo specific primer (neo240F GTCGTGACCCATGGCGATGCCTGCTTG). This multiplex PCR allows amplification from the wild-type locus (if present) from primers +/-F and +/-R giving a 243 bp band. The site for +/-F is deleted in the knockout mice, so this amplification will fail. However, the neo240F primer will amplify a 404 bp band from the targeted locus, in combination with the +/-R primer which anneals to a region just inside the 3' arm. Wild-type samples will exhibit a single 243 bp band, heterozygous DNA samples yield two bands at 243 bp and 404 bp, and the homozygous samples will show only the target specific 404 bp band.

### LacZ Staining

The X gal staining of dissected tissues is performed in the following manner.

Representative tissue slices are made of large organs. Whole small organs and tubes are sliced open, so fixative and stain will penetrate. Tissues are rinsed thoroughly in PBS (phosphate buffered saline) to remove blood or gut contents. Tissues are placed in fixative (PBS containing 2% formaldehyde, 0.2% glutaraldehyde, 0.02% NP40, 1mM MgCl2, Sodium deoxycholate 0.23mM) for 30-45 minutes. Following three 5 minute washes in PBS, tissues are placed in Xgal staining solution (4mM K Ferrocyanide, 4mMKFerncyanide, 2mM MgCl2, 1mg/mlX-gal in PBS) for 18 hours at 30C. Tissues are PBS washed 3 times, postfixed for 24 hours in 4% formaldehyde, PBS washed again before storage in 70% ethanol.

To identify Xgal stained tissues, dehydrated tissues are wax embedded, and 7um section sections cut, counterstained with 0.01% Safranin (9-10 min).

### Behavioural and Neurological Testing

Mice are housed under a 12h lights-12h dark light schedule (lights-on at 6 am) with free access to food and water. Mice (n=12), of mixed sexes, aged 3 to 4 months old, are submitted to behavioural testing during the morning, between 10h and 13h to avoid any differential circadian effect on the test results.

### Example 2. Expression of Recombinant BACH Protein

Recombinant BACH is expressed and purified. Two systems are used for expression.

### pTOPO-Echo Donor Based Construct

A polynucleotide having the sequence shown in SEQ ID NO: 6 is obtained from the mouse BACH nucleic acid sequence (SEQ ID NO: 5). The SEQ ID NO: 6 polynucleotide is cloned into a pTOPO-Echo Donor vector module (Invitrogen pUniV5/His Cat# ET001-10). Transfection of the resulting construct into a host strain and induction of expression (according to the manufacturer's instructions) yields a fusion protein having the sequence of SEQ ID NO: 7.

The fusion polypeptide SEQ ID NO: 7 contains a C terminal V5 tag (residues 379 to 392) and His tag (residues 393 to 398) to aid detection and purification.

### pcDNA 3.1A Myc/His Based Construct

A polynucleotide having the sequence shown in SEQ ID NO: 8 is amplified by PCR using the oligonucleotide primers AAATATAAGGATCCAGACGATGTTAGCCAACAGCTCC and TTCGTGAATTCGAGGGCGGAATCCTGGGGACACTG to incorporate new restriction sites, BamHI and EcoRI at the 5-prime and 3-prime ends of BACH. This is then digested and ligated into similarly digested pcDNA 3.1A Myc/His (Invitrogen Cat# V800-20) to incorporate a novel Kozak consensus sequence (residues 1 to 5 of SEQ ID NO: 8) at the 5' end to improve levels of expression in mammalian cells.

The resulting construct is used for high level expression in CHO-K1 cells, and other mammalian cell lines, under the control of the cmv promoter to yield a fusion polypeptide with C terminal Myc tag (single underline) and His tag (double underline) to aid detection and purification. The resultant expressed fusion polypeptide has a sequence shown in SEQ ID NO: 9.

### Introduction of Construct into Cells

The expression vector is introduced to the cells by lipofection (using Fugene-6 from Roche, Cat# 1 814 433).

Both transient and stable transfection of these cells is achieved. In transient expression the cells are transfected by lipofection using a large amount of vector which results in a short-lived fast expression of the the protein. In a stable transfection, the vector, which includes a selectable marker for neomycin resistance becomes stably integrated into the genome of the host cell resulting in a long lived cell line with a high expression level of BACH.

Cells expressing recombinant BACH are used for assay development, antibody production, and other purposes as described.

### Expression in Other Host Cells

The recombinant/fusion clone SEQ ID NO: 6 is recombined into a pBAD-Thio-E fector (Invitrogen Cat# ET100-01) for high level bacterial expression under control of the *ara*BAD promoter, using a Cre/Lox mediated recombination system.

The recombinant/fusion clone SEQ ID NO: 6 is recombined into a pBlueBac 4.5E (Invitrogen Cat# ET310-01), using a Cre/Lox mediated recombination system, for subsequent recombination into Baculovirus expression systems. Recombination into MaxBac (Invitrogen Cat# K875-02) for high level expression in SF9 and other insect cell lines.

The recombinant/fusion clone SEQ ID NO: 6 is recombined into pcDNA 3.1-E (Invitrogen Cat# ET400-01), using a Cre/Lox mediated recombination system, for high level expression in CHO-K1 (Chinese Hamster Ovary) cells, and other mammalian cell lines, under the control of the *cmv* promoter.

### Example 3. Anti-BACH Antibodies

Monoclonal and polyclonal antibodies which react with BACH protein are produced.

### Anti-Peptide Antibodies

The peptide_CRYRDLEVRL, corresponding to amino acids 165-174 of the full-length BACH polypeptide, is synthesised. The synthethic peptide is injected into sheep to raise polyclonal anti-peptide antibodies. The antibodies are purified by standard methods, and are found to bind to BACH protein in Western blots and ELISA assays.

Injection into rabbits results in rabbit anti-peptide antibodies, which are found to bind to BACH protein in Western blots and ELISA assays.

### Whole Cell Preparations as Antigens

Complete cells expressing a BACH fusion protein, as described above, are used as a source of BACH epitopes in the immunisation. Injection of such cells into sheep results in antibodies which are found to bind to BACH protein in Western blots and ELISA assays. Complete cells are also injected into mice and rabbits for production of anti-BACH antibodies from these organisms.

Complete cells which express the BACH protein in its native form are also used as antigens for injection of sheep, mice and rabbits for the production of polyclonal antibodies.

### Partially Purified Antigens

Crude fractions of cells expressing BACH as a native or fusion protein are made. Membrane fractions are found to include BACH protein and are used to inject sheep for production of anti-BACH antibodies. These antibodies are found to react to BACH protein in Western blots and ELISA assays. Similarly, injection of mice and rabbits results in anti-BACH antibodies which react to BACH protein.

### Purified Antigens

The BACH protein is produced in its native form or as a fusion by the methods described above. BACH protein is purified using affinity chromatography, using the appropriate purification procedure. For example, nickel agarose resins are used to purify His-tagged fusion proteins. If necessary, the fusion protein is cleaved using specific proteases to yield the native protein. Both the tagged polypeptide and the purified cleaved product are used to raise antibodies in a variety of species including sheep, rabbits, mice and goats. These antibodies are found to react to BACH protein.

### Example 4. Expression of BACH in the Dorsal Root Ganglion and the Trigeminal Ganglion

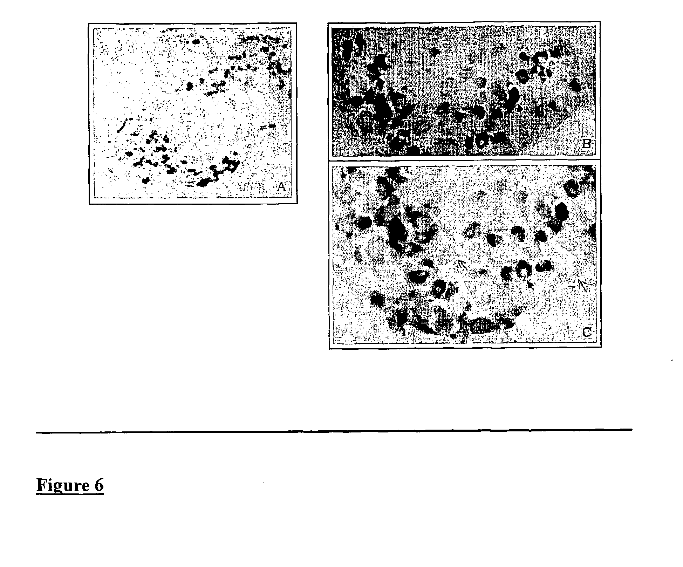

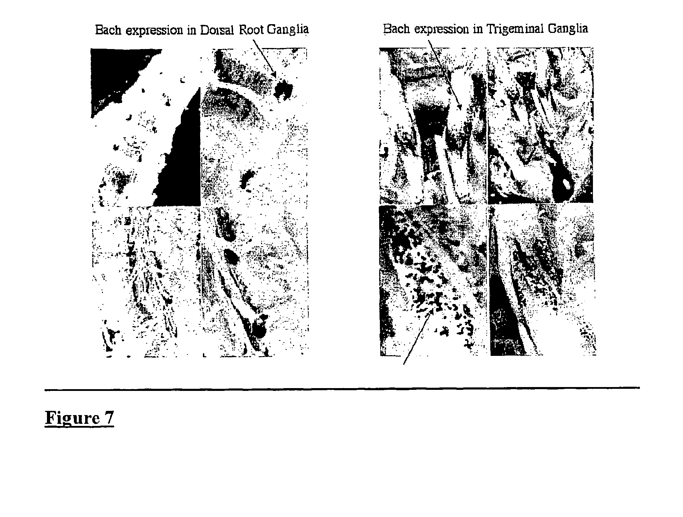

BACH expression is detected in the dorsal root ganglion and the trigeminal ganglion, as shown in Figures 6 and 7.

The trigeminal nerve is the sensory supply to the face, the greater part of the scalp, the teeth, the oral and nasal cavities, the dura matter and the cerebral blood vessels.

It gives the motor supply to the masticatory muscles, and contains proprioceptive fibers from the masticatory and probably the extraocular and fascial muscles. The ophtalmic nerve, where most of the staining was observed, is wholly sensory. It supplies the eyeball, lacrimal gland and conjunctiva, part of the nasal mucosa and the skin of the nose, eyelids, forehead and part of the scalp. Fibres joining the trigeminal from the facial are afferent and arise largely from facial musculature, a minority being proprioceptive, the majority pain.

Lac Z expression is observed on the dorsal surface of the ganglion itself (therefore not on the motor division of the ganglion), with an extension towards the opthalmic and mandibular/maxillary divisions. No staining is observed in the opthalmic, mandibular and maxillary nerves themselves.

Staining in the tongue, the nasal region, the conjuctiva, harderian gland, all are consistent with the trigeminal ganglion's inervation processes. They also favour a role for BACH in pain/sensitivity of the face. Therefore, BACH mutants have modified sensitivity of the face, sense of smell, of taste, or audition.

Examination of the expression pattern and analgesia phenotypic data of the mutant mice show that BACH is a target for potential treatment of trigeminal neuralgia as well as some type of migraine. Orofacial pain is a consequence of trigeminal neuralgia, in which paroxysmal pain radiates over one, or two divisions of the trigeminal nerve. The opthalmic division is rarely affected. Drug treatment is usually effective but if it fails surgical treatment is used. None of these surgical treatments has proved satisfactory. No specific drug has been developed yet.

### Example 5. (reference example) Expression of BACH in the Vestibulocochlear Nerve

Staining is also observed in cranial nerve number 8 (vestibulocochlear) (as it comes out from the skull). P2X receptors are expression in this nerve (Xiang 19991).

This nerve contains 2 major sets of afferent fibers transmitting impulses from the innear ear to the brain. Lesions to this nerve may cause imbalance. We actually observe several mutants inbred mice having difficulty during the wire balance manoeuvre test as well as walking backwards, which is an abnormal hallucinatory behaviour (mice walk backward result from an apparent hallucination of sliding down an inclined plane), and this was much worse when the mice where submitted to the visual cliff test, probably because the chequered pattern accentuated the hallucination.

### Example 6. (reference example) Expression of BACH in the Cerebellum

LacZ staining in cerebellum of the-/-mice was detected in a distinct layer of the cerebellum. The cerebellum links some of the major sensory and motor areas of the CNS. Indeed its main function is for motor learning and reflex modification. The output of the cerebellum is almost entirely to areas of the brain that control movement. Consequently, the rotarod tests that show the-/-mice have improved motor learning and function would almost certainly have altered signalling in the cerebellum. This is supported by the observation of discreet staining in the cerebellum. Application of PPADS, the P2 antagonists to mice resulted in aggressiveness, hyporeactivity and immobility indicating that these receptors may be involved in age related impaired movement disorders.
Although it is believed that P2X receptors are also involved.

### Example 7. (reference example) Analysis of Blood Samples from BACH Knock-Out Mice

Analysis of blood samples taken from mutant BACH animals shows an increased level of circulating cholesterol. This coupled with the expression seen in the liver, gall bladder and in fat around the heart may imply a use for the BACH protein in the development of therapeutics to treat hypercholesterolaemia, dislipdaemias, obesity or drugs to affect energy regulation.

Finally, by comparing with the role and localisation of purine receptors (e. g. P2Y12 regulate mucine expression on the conjuctiva), BACH receptors may also be employed as potential targets for developing drug relating to dry-eye disorders, cystic fibrosis, hyperactive bladder, hypercholesterolaemia, dislipdaemias and obesity.

### Example 8. (reference example) Motor Control and Balance in BACH Knock-Out Mouse

BACH protein has a role in motor co-ordination and balance that can be demonstrated by their performance on the Rotarod apparatus.

Motor co-ordination and balance are measured by performance on the rotarod. We use an accelerating Rotarod (Ugo Basile, Linton instruments, Jones and Roberts 19681).
We also use the Rotarod to assess motor learning by repeating the task over several days.

Mice are placed on the Rotarod, which accelerates at a constant rate from 4 to 40 rpm in 5 in. As the Rotarod reaches higher speed, the mice often grip the Rotarod and hang on for a full rotation (i. e. passive rotation). The time at which the mouse makes one full rotation is recorded. Mice are left on the Rotarod after the first passive rotation and allowed to perform the rest of the test until they drop from the rod. Mice are given 3 trials on three consecutive days.

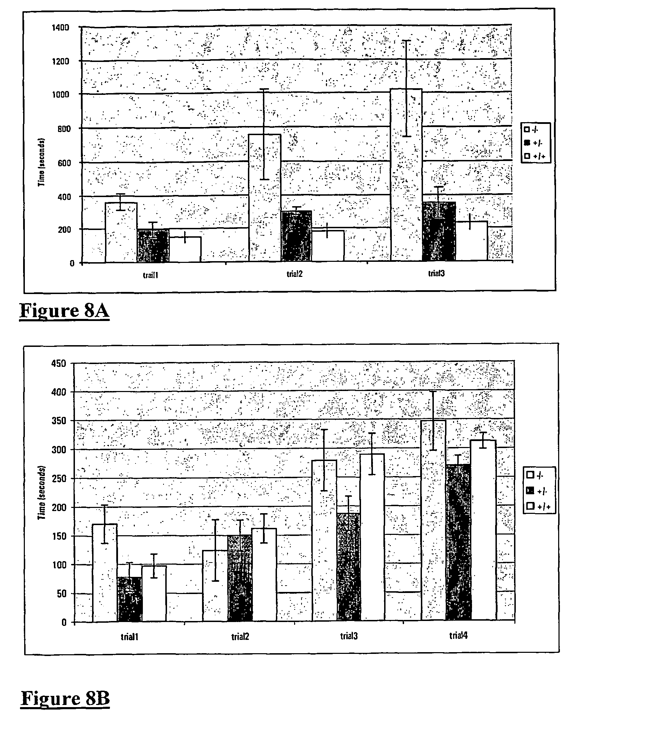

Rotarod testing reveals better performance in mutants than wild type animals in both the outbred and inbred background (p=0. 08). The outbred mutants also shown better motor learning abilities (Figure 8A) (P<0.001). However, this was not observed in inbred animals (Figure 8B).

### Example 9. (reference example) Visual Ability, Anxiety and Mobility in BACH Knock-Out Mouse

Tests for visual ability, anxiety and mobility in a BACH knock-out mouse are conducted using a visual cliff test.

The Visual cliff was developed by Fox (19651) and provides a measure of gross visual ability. It evaluates the ability of the mouse to see the drop-off at the edge of a horizontal surface. Time for the animal to move one placed on the cliff (latency) is a measure of anxiety.

A Perspex box is built with a horizontal plane connected to a vertical drop of 0. 5m. A black and white chequerboard pattern accentuates the vertical drop-out. A sheet of clear Perspex is placed across the top horizontal cliff, extending over the top, thus there is the visual appearance of a cliff, but in fact the Perspex provides a solid horizontal surface.

Each mouse is given 10 trials on the visual cliff. A trial consists of placing the mouse on the centre'ridge'and noting the time taken from the animal to move off the 'ridge' (latency) and recording the side on which the mouse stepped. The result is considered positive when the animal chooses to walk on the chequered"safc"side and avoids the cliff and negative result for the other way round. After 5 trials the box is turned through 180 degrees and a further 5 trials given. This is done to eliminate the variable of the position of the observer. The platform is cleaned between each animal.

**Table 2: Results of BACH (outbred) visual cliff test**

| genotype | average latency | positive | negative |
|---|---|---|---|
| -/- | 79.25 ± 31.29 | 78 ± 7.35% | 22 ± 7.35% |
| +/- | 71.82 ± 24.89 | 55 ± 13.23% | 45 ± 13.53% |
| +/+ | 30.54 ± 10.05 | 67.5 ± 8.54% | 32.5 ± 8.54% |

**Table 3: Results of BACH (inbred) visual cliff tests**

| genotype | average latency | positive | negative |
|---|---|---|---|
| -/- | 51.51 ± 11.78 | 72.9 ± 8.7 % | 27.14 ± 8.65% |
| +/- | 23.82 ± 11.22 | 75 ± 9.6% | 25 ± 9.6% |
| +/+ | 18.05 ± 5.43 | 75 ± 10.4% | 25 ± 10.4% |

Latency ranges from 1 second to 7 min and there is a trend towards a longer latency in mutants (P=0.059). Wild type and mutant animals perform relatively well on the task, with consistent positive results. In heterozygous animals however, results are only positive in 55% of the trials, which is consistent with the kind of results observed with blind mice (Fox, 1965¹) (Table 2). However, there are at least two alternative explanations for this: Firstly, to avoid impairing other tasks, we did not remove the vibrissaes in our mice, and mice are known to use these to navigate. Secondly, we observe that some mice seem somewhat "nervous" during the visual cliff test, displaying either freezing behaviour or alternatively trying to escape the hand of the observer regardless of the cliff whilst being lowered onto the ridge. Although we did not find any significant difference between genotypes in the locomotor behaviour recorded in the arena or the struggle during handling during the Shirpa screen, this could have affected the responses.

As can be seen from the above Tables 2 and 3, BACH mutants have a longer latency period in the visual cliff test when compared to wild type mice, which indicates among other conditions a movement related disorder.

BACH mutants also demonstrate retropulsion (walking backwards). This is accentuated when placed in the visual cliff arena believed to be due to the hallucination of falling forward. This indicates that loss of functional BACH results in movement disorders and dyskinesias.

### Example 10. Tests for Sensitivity to External Stimuli and Pain (Analgesia Testing) in BACH Knock-Out Mouse: Paw Pressure Test

BACH mutants are seen to be less sensitive to touch and pain stimuli using a range of tests. The tests in this and the following four Examples assess both neuropathic and inflammatory pain.

Skin sensitivity is assessed by applying pressure on the hindpaw with a sharpened dowel rod whilst the animal is resting on a grid. Responses are graded as follow: 0 = no withdrawal; 1 =slow withdrawal of the paw; 2 = medium withdrawal of the paw; 3 = fast withdrawal of the paw.

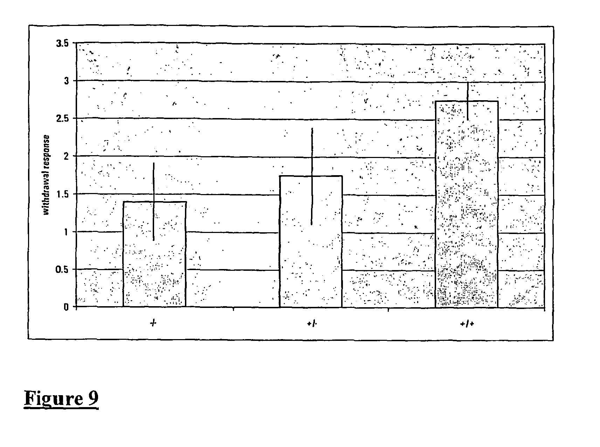

Figure 9 shows results of the paw pressure test. Using the paw pressure test BACH mutants are seen to be less sensitive to pressure and pain than their wild-type counterparts.

### Example 11. Tests for Sensitivity to External Stimuli and Pain (Analgesia Testing) in BACH Knock-Out Mouse: Tail Flick Test

A tail flick analgesia test is performed using a Tail-Flick Analgesia Meter. This equipment provides an easy to use method to determine pain sensitivity accurately and reproducibly in rodents (D'Amour and Smith, 1941). The instrument has a shutter-controlled lamp as a heat source. The lamp is located below the animal to provide a less confining environment. Tail flick is detected by the automatic detection circuitry, which leaves the user's hands free to handle the animal. The animal is restrained in a ventilated tube and its tail placed on a sensing groove on top of the equipment.

Activation of an intense light beam to the tail through opening of the shutter results in discomfort at some point when the animal will flick its tail out of the beam. In the automatic mode a photo-detector detects the tail motion causing the clock to stop and the shutter to close. The total time elapsed between the shutter opening and the animal's reaction is recorded.

Responses of mutant transgenic mice are compared with age and sex matched wild-type mice. A single animal may be subjected to different heat settings to produce an increase in tail temperature no greater than 55°C.

Using the tail flick test BACH mutants are shown to be less sensitive to heat induced pain than their wild-type counterparts and withdraw their tails after a longer time period of exposure to the heat source.

### Example 12. Tests for Sensitivity to External Stimuli and Pain (Analgesia Testing) in BACH Knock-Out Mouse: Formalin Test

The formalin test measures the response to a noxious substances injected into a hind paw. A volume of 20µl of a 1% formalin solution is injected through a fine gauge needle subcutaneously into the dorsal surface of one hindpaw. Licking and biting the hindpaw is quantitated as cumulative number of seconds engaged in the behaviours. A rating scale is used: 1= the formalin injected paw rests lightly on the floor bearing less weight; 2= the injected paw is elevated; 3= the injected paw is licked, bitten or shaken.

Two phases of responses are seen in the formalin test. Phase 1 begins immediately after injection and lasts about 10 mins, representing the acute burst of activity from pain fibres. Phase two begins about 20 mins after injection and continues for about one hour. This phase appears to represent responses to tissue damage, including inflammatory hyperalgesia.

Using the formalin test BACH mutants are shown to be less sensitive to inflammatory pain and show a reduced severity of response in the formalin test when compared to wild type animals.

### Example 13. Tests for Sensitivity to External Stimuli and Pain (Analgesia Testing) in BACH Knock-Out Mouse: Von Frey Hair Test

A test for touch, which is used to measure pain thresholds, employs von Frey hairs. These hairs are a set of very fine gauge calibrated wires. Withdrawal threshold to mechanical stimulation is measured. The animal stands on an elevated platform in which the surface is a wide gauge wire mesh. The Von Frey hair is inserted from below, up through the holes in the mesh, to poke the undersurface of the hindpaw. At threshold, the mouse responds by flicking its paw away from the hair, generally followed by raising the paw, licking the paw, and or vocalisation. Mechanical withdrawal threshold is defined as the minimum gauge wire stimulus that elicits withdrawal reactions in two out of three consecutive trials.

In the Von Frey hair test for touch, mutant BACH mice are seen to have a higher mechanical withdrawal threshold demonstrating that they are less sensitive to the stimuli.

### Example 14. Tests for Sensitivity to External Stimuli and Pain (Analgesia Testing) in BACH Knock-Out Mouse: Urogenital Function

BACH is expressed in the bladder and by measuring the frequency of micturation and the volume of fluid released. Loss of this protein is shown to result in abnormal bladder motility. BACH mutants have hypoactive bladder urinating less often but releasing a larger volume and suffer urinary retention. It is likely that BACH plays a role in erectile dysfunction and the control of motor fibres in the prostate.

### Example 15. Identification of BACH Regulatory Elements

During evolution genes undergo sequence divergence. Sequences that affect the function of a gene tend to be better conserved and these include not only coding sequences but also promoter control element and enhancers. By identification of homologues and orthologues of BACH in multiple species and comparison of the promoter regions such control elements and enhancers are located.

To find human BACH control elements and enhancers we compare the promoter regions from human BACH to the promoter region of BACH from the pufferfish (*Takifugu rubripes*) using Blast type alignments. Conserved patches of sequence are the critical elements that control the level, the timing and the location of BACH gene expression.

Human populations and disease cohorts are then screened for polymorphisms and in the control regions identified in this manner. Mutations, such as SNPs, in these regions will affect the control of BACH gene expression and will cause conditions such as BACH associated diseases. Similar mutations are generated in the promoter sequences and used to assess their effect on either BACH or a reporter gene expression in cell lines or transgenic mice. Polymorphisms that are shown to have a functional effect are then used in diagnostic screens for BACH specific diseases conditions outlined.

### REFERENCES

Berchtold S, Ogilvie AL, Bogdan C, Muhl-Zurbes P, Ogilvie A, Schuler G, Steinkasserer A. Human monocyte derived dendritic cells express functional P2X and P2Y receptors as well as ecto-nucleotidases. FEBS Lett. 1999 Sep 24;458(3):424-8.
Burnstock G. Purine-mediated signalling in pain and visceral perception. Trends Pharmacol Sci. 2001 Apr;22(4):182-8.
Burstein, R., Cutrer, F.M. and Yarnitsky, D., (2000 a) The Development of CutaneousAllodynia During a Migraine Attack: Clinical Evidence for the Sequential Recruitment of Spinal and Supraspinal Nociceptive Neurons in Migraine, Brain, 123(8), 1703-1709.
Burstein, R., Yarnitsky, D., Goor-Aryeh, I., Ransil, B.J. and Bajwa, Z., (2000 b) An Association Between Migraine and Cutaneous Allodynia, Ann. Neurol. 47 (5) 614-624.
Cockayne DA, Hamilton SG, Zhu QM, Dunn PM, Zhong Y, Novakovic S, Malmberg AB, Cain G, Berson A, Kassotakis L, Hedley L, Lachnit WG, Burnstock G, McMahon SB, Ford AP. Urinary bladder hyporeflexia and reduced pain-related behaviour in P2X3-deficient mice. Nature. 2000 Oct 26;407(6807):1011-5.
Evans MJ, Kaufman MH. Establishment in culture of pluripotential cells from mouse embryos. Nature. 1981 Jul 9;292(5819):154-6.
Foster CJ, Prosser DM, Agans JM, Zhai Y, Smith MD, Lachowicz JE, Zhang FL, Gustafson E, Monsma FJ Jr, Wiekowski MT, Abbondanzo SJ, Cook DN, Bayne ML, Lira SA, Chintala MS. Molecular identification and characterization of the platelet ADP receptor targeted by thienopyridine antithrombotic drugs. J Clin Invest. 2001 Jun;107(12):1591-8.
Jumblatt JE, Jumblatt MM. Regulation of ocular mucin secretion by P2Y2 nucleotide receptors in rabbit and human conjunctiva. Exp Eye Res. 1998 Sep;67(3):341-6.
Jones BJ and Roberts DJ. The quantitative measurement of motor incoordination in naïve mice using and accelerating rotarod. (1968) J Pharm Pharmac 20, 302-304.
Kaplan MH, Smith DI, Sundick RS. Identification of a G protein coupled receptor induced in activated T cells. J Immunol. 1993 Jul 15;151(2):628-36.
Leon C, Hechler B, Freund M, Eckly A, Vial C, Ohlmann P, Dierich A, LeMeur M, Cazenave JP, Gachet C. Defective platelet aggregation and increased resistance to thrombosis in purinergic P2Y(1) receptor-null mice. J Clin Invest. 1999 Dec;104(12):1731-7.
Li Q, Schachter JB, Harden TK, Nicholas RA. The 6H1 orphan receptor, claimed to be the p2y5 receptor, does not mediate nucleotide-promoted second messenger responses. Biochem Biophys Res Commun. 1997 Jul 18;236(2):455-60.
Mulryan K, Glitterman DP, Lewis CJ, Vial C, Leckie BJ. Reduced vas deferens contraction and male infertility in mice lacking P2X1 receptors. Nature 2000, 403:86-89.
Nakamura F, Strittmatter SM. P2Y1 purinergic receptors in sensory neurons: contribution to touch-induced impulse generation. Proc Natl Acad Sci U S A. 1996 Sep 17;93(19):10465-70.
Noone PG, Hamblett N, Accurso F, Aitken ML, Boyle M, Dovey M, Gibson R, Johnson C, Kellerman D, Konstan MW, Milgram L, Mundahl J, Retsch-Bogort G, Rodman D, Williams-Warren J, Wilmott RW, Zeitlin P, Ramsey B. Safety of aerosolized INS 365 in patients with mild to moderate cystic fibrosis: results of a phase I multi-center study. Pediatr Pulmonol. 2001 Aug;32(2):122-8.
Pintor J, Diaz-Hernandez M, Gualix J, Gomez-Villafuertes R, Hernando F, Miras-Portugal MT. Diadenosine polyphosphate receptors. from rat and guinea-pig brain to human nervous system. Pharmacol Ther. 2000 Aug-Sep;87(2-3):103-15.
Rogers DC, Fisher EM, Brown SD, Peters J, Hunter AJ, Martin JE. Behavioral and functional analysis of mouse phenotype: SHIRPA, a proposed protocol for comprehensive phenotype assessment. Mamm Genome (1997) 8(10), 711-713.
Russ AP, Sigrid W, Colledge WH, Aparicio SAJR, Carlton MBL, Pearce JJ, Barton SC, Surani MA, Ryan K, Nehls MC, Wilson V and Evans MJ. 2000. Eomesodermin is required for trophoblast development and mesoderm formation in the mouse embryo. Nature 404(6773):95-9.
Souslova V, Cesare P, Ding Y, Akopian AN, Stanfa L, Suzuki R, Carpenter K, Dickenson A, Boyce S, Hill R, Nebenuis-Oosthuizen D, Smith AJ, Kidd EJ, Wood JN. Warm-coding deficits and aberrant inflammatory pain in mice lacking P2X3 receptors. Nature. 2000 Oct 26;407(6807):1015-7.
Storey F. The P2Y12 receptor as a therapeutic target in cardiovascular disease. Platelets. 2001 Jun; 12 (4): 197-209. Review. Kugelgen I, Wetter A. Molecular pharmacology of P2Y-receptors. Naunyn Schmiedebergs Arch Pharmacol. 2000 Nov; 362 (4-5): 310-23.13.
Von Kugelgen I, Wetter A. Molecular pharmacology of P2Y-receptors. Naunyn Schmiedebergs Arch Pharmacol. 2000 Nov ; 362 (4-5): 310-23.
Webb TE, Kaplan MG, Bamard EA. Identification of 6H1 as a P2Y purinoceptor: P2Y5. Biochem Biophys Res Commun. 1996 Feb 6; 219 (1): 105-10.
Xiang Z, Bo X, Burnstock G. P2X receptor immunoreactivity in the rat cochlea, vestibular ganglion and cochlear nucleus. Hear Res. 1999 Feb; 128 (1-2): 190-6.

Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the claims.

### SEQUENCE LISTING

<110> Paradigm Therapeutics Limited
<120> Receptor
<130> P9561WO
<150> GB0027170.0
   <151> 2000-11-07
<150> US 60/248,411
   <151> 2000-11-14
<160> 9
<170> SeqWin99, version 1.02
<210> 1
   <211> 1668
   <212> DNA
   <213> Homo sapiens
<400> 1
   ctcccaatcc cactttggca cgatgttagc caacagctcc tcaaccaaca gttctgttct    60
   cccgtgtcct gactaccgac ctacccaccg cctgcacttg gtggtctaca gcttggtgct   120
   ggctgccggg ctccccctca acgcgctagc cctctgggtc ttcctgcgcg cgctgcgcgt   180
   gcactcggtg gtgagcgtgt acatgtgtaa cctggcggcc agcgacctgc tcttcaccct   240
   ctcgctgccc gttcgtctct cctactacgc actgcaccac tggcccttcc ccgacctcct   300
   gtgccagacg acgggcgcca tcttccagat gaacatgtac ggcagctgca tcttcctgat   360
   gctcatcaac gtggaccgct acgccgccat cgtgcacccg ctgcgactgc gccacctgcg   420
   gcggccccgc gtggcgcggc tgctctgcct gggcgtgtgg gcgctcatcc tggtgtttgc   480
   cgtgcccgcc gcccgcgtgc acaggccctc gcgttgccgc taccgggacc tcgaggtgcg   540
   cctatgcttc gagagcttca gcgacgagct gtggaaaggc aggctgctgc ccctcgtgct    600
   gctggccgag gcgctgggct tcctgctgcc cctggcggcg gtggtctact cgtcgggccg   660
   agtcttctgg acgctggcgc gccccgacgc cacgcagagc cagcggcggc ggaagaccgt   720
   gcgcctcctg ctggctaacc tcgtcatctt cctgctgtgc ttcgtgccct acaacagcac   780
   gctggcggtc tacgggctgc tgcggagcaa gctggtggcg gccagcgtgc ctgcccgcga   840
   tcgcgtgcgc ggggtgctga tggtgatggt gctgctggcc ggcgccaact gcgtgctgga   900
   cccgctggtg tactacttta gcgccgaggg cttccgcaac accctgcgcg gcctgggcac   960
   tccgcaccgg gccaggacct cggccaccaa cgggacgcgg gcggcgctcg cgcaatccga  1020
   aaggtccgcc gtcaccaccg acgccaccag gccggatgcc gccagtcagg ggctgctccg  1080
   accctccgac tcccactctc tgtcttcctt cacacagtgt ccccaggatt ccgccctctg  1140
   aacacacatg ccattgcgct gtccgtgccc gactcccaac gcctctcgtt ctgggaggct  1200
   tacagggtgt acacacaaga aggtgggctg ggcacttgga cctttgggtg gcaattccag  1260
   cttagcaacg cagaagagta caaagtgtgg aagccagggc ccagggaagg cagtgctgct  1320
   ggaaatggct tctttaaact gtgagcacgc agagcacccc ttctccagcg gtgggaagtg  1380
   atgcagagag cccacccgtg cagagggcag aagaggacga aatgcctttg ggtgggcagg  1440
   gcattaaact gctaaaagct ggttagatgg aacagaaaat gggcattctg gatctaaacc  1500
   gccacagggg cctgagagct gaagagcacc aggtttggtg gacaaagcta ctgagatgcc  1560
   tgttcatctc ctgacttctg tctaggctca tggatgccac cccctttcat ttcggcctag  1620
   gcttcccctg ctcaccactg aggcctaata caagagttcc aaaaaaaa               1668
<210> 2
   <211> 1119
   <212> DNA
   <213> Homo sapiens
<400> 2
   atgttagcca acagctcctc aaccaacagt tctgttctcc cgtgtcctga ctaccgacct 60
   acccaccgcc tgcacttggt ggtctacagc ttggtgctgg ctgccgggct ccccctcaac 120
   gcgctagccc tctgggtctt cctgcgcgcg ctgcgcgtgc actcggtggt gagcgtgtac 180
   atgtgtaacc tggcggccag cgacctgctc ttcaccctct cgctgcccgt tcgtctctcc 240
   tactacgcac tgcaccactg gcccttcccc gacctcctgt gccagacgac gggcgccatc 300
   ttccagatga acatgtacgg cagctgcatc ttcctgatgc tcatcaacgt ggaccgctac 360
   gccgccatcg tgcacccgct gcgactgcgc cacctgcggc ggccccgcgt ggcgcggctg 420
   ctctgcctgg gcgtgtgggc gctcatcctg gtgtttgccg tgcccgccgc ccgcgtgcac 480
   aggccctcgc gttgccgcta ccgggacctc gaggtgcgcc tatgcttcga gagcttcagc 540
   gacgagctgt ggaaaggcag gctgctgccc ctcgtgctgc tggccgaggc gctgggcttc 600
   ctgctgcccc tggcggcggt ggtctactcg tcgggccgag tcttctggac gctggcgcgc 660
   cccgacgcca cgcagagcca gcggcggcgg aagaccgtgc gcctcctgct ggctaacctc 720
   gtcatcttcc tgctgtgctt cgtgccctac aacagcacgc tggcggtcta cgggctgctg 780
   cggagcaagc tggtggcggc cagcgtgcct gcccgcgatc gcgtgcgcgg ggtgctgatg 840
   gtgatggtgc tgctggccgg cgccaactgc gtgctggacc cgctggtgta ctactttagc 900
   gccgagggct tccgcaacac cctgcgcggc ctgggcactc cgcaccgggc caggacctcg 960
   gccaccaacg ggacgcgggc ggcgctcgcg caatccgaaa ggtccgccgt caccaccgac 1020
   gccaccaggc cggatgccgc cagtcagggg ctgctccgac cctccgactc ccactctctg 1080
   tcttccttca cacagtgtcc ccaggattcc gccctctga 1119
<210> 3
   <211> 372
   <212> PRT
   <213> Homo sapiens
<400> 3
<210> 4
   <211> 1467
   <212> DNA
   <213> Mus musculus
<400> 4
   atggatggtg aaagatggcc tgaagagcct ttgaggccaa gaaaggactc aagaggccag    60
   tggtcagcca agtgtttagg ctacggttct gtttctcata gctttacttt tggcttgcct   120
   tcccactcca ggtctccact gctgaagaag ttgcagcacc aagggcttcc agccttggcc   180
   agtcatctgc ccctgaggtc acagcccacg tgcccatgtc ttgcttctgc ttgcctgggg   240
   acagagtctg tattgccacc aggctaccac cctggaggtg aaagtcatgc tctgaccttg   300
   ttgttcccta cgatgcctca gactaatttc tcttcccacc tggacatgat gtttgccaat   360
   tcttcagcca acacgacttc taccaacagc tctgtgctcc agtgccctga ctatcgagat   420
   acacatcgtt tgcatatggt ggtctacagc ctggtattgg cgactggtct ccctctcaac   480
   gctctggctc tctgggtctt cctgcgtgta ctgcgcgtac actcagtggt gagcgtgtac   540
   atgtgcaacc tggcagccag cgacttgctc ttcaccctgt cacttcccct gcgcctctcc   600
   tactatgcac agcaccactg gccttttcca ggcttcctgt gccagacgtc gggcgccatc   660
   ttccagatga acatgtacgg cagctgtctc tttctgatgc tcatcaacgt ggaccgctat   720
   gcggccatcg tgcacccgct gagactgcgc cacctacggc ggccccgtgt ggcacggcgg   780
   ctctgcctgg gcgtgtgggc tctcatcctg ctgttcgctg tgcccgccgc ccgcgtgcac   840
   agcccgtccc actgcacgta caagaacatc actgtgcgcc tgtgcttcga gagcttcagc   900
   gatgaactgt ggaagggcag gctgctgccg ctcctgctgc tggccgagat actaggcttt   960
   ctgctgcccc tggcggctgt cgtctattcg tctggcagag tcttctggac actggcgagg  1020
   cccgacgcca ctcagagcca acggcgacgg aagaccgtgc gcctcctgct ggccaatctc  1080
   atcatcttcc tgctgtgctt cgtgccctat aactccacgc tggctgtata tgggttgcta  1140
   cgggccaact tggtgaagaa cagtattcag gaccgcgatc aggtgcgcgg ggtgctgatg  1200
   ataatggtgc tgctggccgg cgccaactgc gtgctggatc cactggttta ctacttcagt  1260
   gccgagggtt tccgtaacac ccttcgcaac ctgggcgccc cgctgaatac caggcctttg  1320
   gctaccaatg ggctgcaggc cgtgctcacc gaactaccct cagaaagcac ccaaaacact  1380
   gggcaggatg ccacaagtca ggttctactc cagcctgcca ctctgggtac acccccggac  1440
   aactgctccc aggattcggc tctctga                                      1467
<210> 5
   <211> 488
   <212> PRT
   <213> Mus musculus
<400> 5
   Met Asp Gly Glu Arg Trp Pro Glu Glu Pro Leu Arg Pro Arg Lys Asp
   1               5                   10                  15
   Ser Arg Gly Gln Trp Ser Ala Lys Cys Leu Gly Tyr Gly Ser Val Ser
               20                  25                  30
   His Ser Phe Thr Phe Gly Leu Pro Ser His Ser Arg Ser Pro Leu Leu
           35                  40                  45
   Lys Lys Leu Gln His Gln Gly Leu Pro Ala Leu Ala Ser His Leu Pro
       50                  55                  60
   Leu Arg Ser Gln Pro Thr Cys Pro Cys Leu Ala Ser Ala Cys Leu Gly
   65                  70                  75                  80
   Thr Glu Ser Val Leu Pro Pro Gly Tyr His Pro Gly Gly Glu Ser His
                   85                  90                  95
   Ala Leu Thr Leu Leu Phe Pro Thr Met Pro Gln Thr Asn Phe Ser Ser
               100                 105                 110
   His Leu Asp Met Met Phe Ala Asn Ser Ser Ala Asn Thr Thr Ser Thr
           115                 120                 125
   Asn Ser Ser Val Leu Gln Cys Pro Asp Tyr Arg Asp Thr His Arg Leu
       130                 135                 140
   His Met Val Val Tyr Ser Leu Val Leu Ala Thr Gly Leu Pro Leu Asn
   145                 150                 155                 160
   Ala Leu Ala Leu Trp Val Phe Leu Arg Val Leu Arg Val His Ser Val
                   165                 170                 175
   Val Ser Val Tyr Met Cys Asn Leu Ala Ala Ser Asp Leu Leu Phe Thr
               180                 185                 190
   Leu Ser Leu Pro Leu Arg Leu Ser Tyr Tyr Ala Gln His His Trp Pro
           195                 200                 205
   Phe Pro Gly Phe Leu Cys Gln Thr Ser Gly Ala Ile Phe Gln Met Asn
       210                 215                 220
   Met Tyr Gly Ser Cys Leu Phe Leu Met Leu Ile Asn Val Asp Arg Tyr
   225                 230                 235                 240
   Ala Ala Ile Val His Pro Leu Arg Leu Arg His Leu Arg Arg Pro Arg
                   245                 250                 255
   Val Ala Arg Arg Leu Cys Leu Gly Val Trp Ala Leu Ile Leu Leu Phe
               260                 265                 270
   Ala Val Pro Ala Ala Arg Val His Ser Pro Ser His Cys Thr Tyr Lys
           275                 280                 285
   Asn Ile Thr Val Arg Leu Cys Phe Glu Ser Phe Ser Asp Glu Leu Trp
       290                 295                 300
   Lys Gly Arg Leu Leu Pro Leu Leu Leu Leu Ala Glu Ile Leu Gly Phe
   305                 310                 315                 320
   Leu Leu Pro Leu Ala Ala Val Val Tyr Ser Ser Gly Arg Val Phe Trp
                   325                 330                 335
   Thr Leu Ala Arg Pro Asp Ala Thr Gln Ser Gln Arg Arg Arg Lys Thr
               340                 345                 350
   Val Arg Leu Leu Leu Ala Asn Leu Ile Ile Phe Leu Leu Cys Phe Val
           355                 360                 365
   Pro Tyr Asn Ser Thr Leu Ala Val Tyr Gly Leu Leu Arg Ala Asn Leu
       370                 375                 380
   Val Lys Asn Ser Ile Gln Asp Arg Asp Gln Val Arg Gly Val Leu Met
   385                 390                 395                 400
   Ile Met Val Leu Leu Ala Gly Ala Asn Cys Val Leu Asp Pro Leu Val
                   405                 410                 415
   Tyr Tyr Phe Ser Ala Glu Gly Phe Arg Asn Thr Leu Arg Asn Leu Gly
               420                 425                 430
   Ala Pro Leu Asn Thr Arg Pro Leu Ala Thr Asn Gly Ala Ala Gly Val
           435                 440                 445
   Leu Thr Glu Leu Pro Ser Glu Ser Thr Gln Asn Thr Gly Gln Asp Ala
       450                 455                 460
   Thr Ser Gln Val Leu Leu Gln Pro Ala Thr Leu Gly Thr Pro Pro Asp
   465                 470                 475                 480
   Asn Cys Ser Gln Asp Ser Ala Leu
                   485
<210> 6
   <211> 1197
   <212> DNA
   <213> Mus musculus
<400> 6
   atgttagcca acagctcctc aaccaacagt tctgttctcc cgtgtcctga ctaccgacct 60
   acccaccgcc tgcacttggt ggtctacagc ttggtgctgg ctgccgggct ccccctcaac 120
   gcgctagccc tctgggtctt cctgcgcgcg ctgcgcgtgc actcggtggt gagcgtgtac 180
   atgtgtaacc tggcggccag cgacctgctc ttcaccctct cgctgcccgt tcgtctctcc 240
   tactacgcac tgcaccactg gcccttcccc gacctcctgt gccagacgac gggcgccatc 300
   ttccagatga acatgtacgg cagctgcatc ttcctgatgc tcatcaacgt ggaccgctac 360
   gccgccatcg tgcacccgct gcgactgcgc cacctgcggc ggccccgcgt ggcgcggctg 420
   ctctgcctgg gcgtgtgggc gctcatcctg gtgtttgccg tgcccgccgc ccgcgtgcac 480
   aggccctcgc gttgccgcta ccgggacctc gaggtgcgcc tatgcttcga gagcttcagc 540
   gacgagctgt ggaaaggcag gctgctgccc ctcgtgctgc tggccgaggc gctgggcttc 600
   ctgctgcccc tggcggcggt ggtctactcg tcgggccgag tcttctggac gctggcgcgc 660
   cccgacgcca cgcagagcca gcggcggcgg aagaccgtgc gcctcctgct ggctaacctc 720
   gtcatcttcc tgctgtgctt cgtgccctac aacagcacgc tggcggtcta cgggctgctg 780
   cggagcaagc tggtggcggc cagcgtgcct gcccgcgatc gcgtgcgcgg ggtgctgatg 840
   gtgatggtgc tgctggccgg cgccaactgc gtgctggacc cgctggtgta ctactttagc 900
   gccgagggct tccgcaacac cctgcgcggc ctgggcactc cgcaccgggc caggacctcg 960
   gccaccaacg ggacgcgggc ggcgctcgcg caatccgaaa ggtccgccgt caccaccgac 1020
   gccaccaggc cggatgccgc cagtcagggg ctgctccgac cctccgactc ccactctctg 1080
   tcttccttca cacagtgtcc ccaggattcc gccctaaggg caattcggga gctcggtaag 1140
   cctatcccta accctctcct cggtctcgat tctagccatc atcaccatca ccattga 1197
<210> 7
   <211> 398
   <212> PRT
   <213> Mus musculus
<220>
   <221> BINDING
   <222> 379-392
   <223> C terminal V5 tag
<220>
   <221> BINDING
   <222> 393-398
   <223> His tag
<400> 7
   Met Leu Ala Asn Ser Ser Ser Thr Asn Ser Ser Val Leu Pro Cys Pro
   1               5                   10                  15
   Asp Tyr Arg Pro Thr His Arg Leu His Leu Val Val Tyr Ser Leu Val
               20                  25                  30
   Leu Ala Ala Gly Leu Pro Leu Asn Ala Leu Ala Leu Trp Val Phe Leu
           35                  40                  45
   Arg Ala Leu Arg Val His Ser Val Val Ser Val Tyr Met Cys Asn Leu
       50                  55                  60
   Ala Ala Ser Asp Leu Leu Phe Thr Leu Ser Leu Pro Val Arg Leu Ser
   65                  70                  75                  80
   Tyr Tyr Ala Leu His His Trp Pro Phe Pro Asp Leu Leu Cys Gln Thr
                   85                  90                  95
   Thr Gly Ala Ile Phe Gln Met Asn Met Tyr Gly Ser Cys Ile Phe Leu
               100                 105                 110
   Met Leu Ile Asn Val Asp Arg Tyr Ala Ala Ile Val His Pro Leu Arg
           115                 120                 125
   Leu Arg His Leu Arg Arg Pro Arg Val Ala Arg Leu Leu Cys Leu Gly
       130                 135                 140
   Val Trp Ala Leu Ile Leu Val Phe Ala Val Pro Ala Ala Arg Val His
   145                 150                 155                 160
   Arg Pro Ser Arg Cys Arg Tyr Arg Asp Leu Glu Val Arg Leu Cys Phe
                   165                 170                 175
   Glu Ser Phe Ser Asp Glu Leu Trp Lys Gly Arg Leu Leu Pro Leu Val
               180                 185                 190
   Leu Leu Ala Glu Ala Leu Gly Phe Leu Leu Pro Leu Ala Ala Val Val
           195                 200                 205
   Tyr Ser Ser Gly Arg Val Phe Trp Thr Leu Ala Arg Pro Asp Ala Thr
       210                 215                 220
   Gln Ser Gln Arg Arg Arg Lys Thr Val Arg Leu Leu Leu Ala Asn Leu
   225                 230                 235                 240
   Val Ile Phe Leu Leu Cys Phe Val Pro Tyr Asn Ser Thr Leu Ala Val
                   245                 250                 255
   Tyr Gly Leu Leu Arg Ser Lys Leu Val Ala Ala Ser Val Pro Ala Arg
               260                 265                 270
   Asp Arg Val Arg Gly Val Leu Met Val Met Val Leu Leu Ala Gly Ala
           275                 280                 285
   Asn Cys Val Leu Asp Pro Leu Val Tyr Tyr Phe Ser Ala Glu Gly Phe
       290                 295                 300
   Arg Asn Thr Leu Arg Gly Leu Gly Thr Pro His Arg Ala Arg Thr Ser
   305                 310                 315                 320
   Ala Thr Asn Gly Thr Arg Ala Ala Leu Ala Gln Ser Glu Arg Ser Ala
                   325                 330                 335
   Val Thr Thr Asp Ala Thr Arg Pro Asp Ala Ala Ser Gln Gly Leu Leu
               340                 345                 350
   Arg Pro Ser Asp Ser His Ser Leu Ser Ser Phe Thr Gln Cys Pro Gln
           355                 360                 365
   Asp Ser Ala Leu Arg Ala Ile Arg Glu Leu Gly Lys Pro Ile Pro Asn
       370                 375                 380
   Pro Leu Leu Gly Leu Asp Ser Ser His His His His His His
   385                 390                 395
<210> 8
   <211> 1241
   <212> DNA
   <213> Mus musculus
<220>
   <221> MISC_SIGNAL
   <222> 1-5
   <223> Kozak consensus signal
<400> 8
   agacgatgtt agccaacagc tcctcaacca acagttctgt tctcccgtgt cctgactacc   60
   gacctaccca ccgcctgcac ttggtggtct acagcttggt gctggctgcc gggctccccc  120
   tcaacgcgct agccctctgg gtcttcctgc gcgcgctgcg cgtgcactcg gtggtgagcg  180
   tgtacatgtg taacctggcg gccagcgacc tgctcttcac cctctcgctg cccgttcgtc  240
   tctcctacta cgcactgcac cactggccct tccccgacct cctgtgccag acgacgggcg  300
   ccatcttcca gatgaacatg tacggcagct gcatcttcct gatgctcatc aacgtggacc  360
   gctacgccgc catcgtgcac ccgctgcgac tgcgccacct gcggcggccc cgcgtggcgc  420
   ggctgctctg cctgggcgtg tgggcgctca tcctggtgtt tgccgtgccc gccgcccgcg  480
   tgcacaggcc ctcgcgttgc cgctaccggg acctcgaggt gcgcctatgc ttcgagagct  540
   tcagcgacga gctgtggaaa ggcaggctgc tgcccctcgt gctgctggcc gaggcgctgg  600
   gcttcctgct gcccctggcg gcggtggtct actcgtcggg ccgagtcttc tggacgctgg  660
   cgcgccccga cgccacgcag agccagcggc ggcggaagac cgtgcgcctc ctgctggcta  720
   acctcgtcat cttcctgctg tgcttcgtgc cctacaacag cacgctggcg gtctacgggc  780
   tgctgcggag caagctggtg gcggccagcg tgcctgcccg cgatcgcgtg cgcggggtgc  840
   tgatggtgat ggtgctgctg gccggcgcca actgcgtgct ggacccgctg gtgtactact  900
   ttagcgccga gggcttccgc aacaccctgc gcggcctggg cactccgcac cgggccagga  960
   cctcggccac caacgggacg cgggcggcgc tcgcgcaatc cgaaaggtcc gccgtcacca 1020
   ccgacgccac caggccggat gccgccagtc aggggctgct ccgaccctcc gactcccact 1080
   ctctgtcttc cttcacacag tgtccccagg attccgccct cgaattctgc agatatccag 1140
   cacagtggcg gccgctcgag tctagagggc ccttcgaaca aaaactcatc tcagaagagg 1200
   atctgaatat gcataccggt catcatcacc atcaccattg a                      1241
<210> 9
   <211> 411
   <212> PRT
   <213> Mus musculus
<220>
   <221> BINDING
   <222> 391-400
   <223> C terminal Myc tag
<220>
   <221> BINDING
   <222> 406-411
   <223> His tag
<400> 9
   Met Leu Ala Asn Ser Ser Ser Thr Asn Ser Ser Val Leu Pro Cys Pro
   1               5                   10                  15
   Asp Tyr Arg Pro Thr His Arg Leu His Leu Val Val Tyr Ser Leu Val
               20                  25                  30
   Leu Ala Ala Gly Leu Pro Leu Asn Ala Leu Ala Leu Trp Val Phe Leu
           35                  40                  45
   Arg Ala Leu Arg Val His Ser Val Val Ser Val Tyr Met Cys Asn Leu
       50                  55                  60
   Ala Ala Ser Asp Leu Leu Phe Thr Leu Ser Leu Pro Val Arg Leu Ser
   65                  70                  75                  80
   Tyr Tyr Ala Leu His His Trp Pro Phe Pro Asp Leu Leu Cys Gln Thr
                   85                  90                  95
   Thr Gly Ala Ile Phe Gln Met Asn Met Tyr Gly Ser Cys Ile Phe Leu
               100                 105                 110
   Met Leu Ile Asn Val Asp Arg Tyr Ala Ala Ile Val His Pro Leu Arg
           115                 120                 125
   Leu Arg His Leu Arg Arg Pro Arg Val Ala Arg Leu Leu Cys Leu Gly
       130                 135                 140
   Val Trp Ala Leu Ile Leu Val Phe Ala Val Pro Ala Ala Arg Val His
   145                 150                 155                 160
   Arg Pro Ser Arg Cys Arg Tyr Arg Asp Leu Glu Val Arg Leu Cys Phe
                   165                 170                 175
   Glu Ser Phe Ser Asp Glu Leu Trp Lys Gly Arg Leu Leu Pro Leu Val
               180                 185                 190
   Leu Leu Ala Glu Ala Leu Gly Phe Leu Leu Pro Leu Ala Ala Val Val
           195                 200                 205
   Tyr Ser Ser Gly Arg Val Phe Trp Thr Leu Ala Arg Pro Asp Ala Thr
       210                 215                 220
   Gln Ser Gln Arg Arg Arg Lys Thr Val Arg Leu Leu Leu Ala Asn Leu
   225                 230                 235                 240
   Val Ile Phe Leu Leu Cys Phe Val Pro Tyr Asn Ser Thr Leu Ala Val
                   245                 250                 255
   Tyr Gly Leu Leu Arg Ser Lys Leu Val Ala Ala Ser Val Pro Ala Arg
               260                 265                 270
   Asp Arg Val Arg Gly Val Leu Met Val Met Val Leu Leu Ala Gly Ala
           275                 280                 285
   Asn Cys Val Leu Asp Pro Leu Val Tyr Tyr Phe Ser Ala Glu Gly Phe
       290                 295                 300
   Arg Asn Thr Leu Arg Gly Leu Gly Thr Pro His Arg Ala Arg Thr Ser
   305                 310                 315                 320
   Ala Thr Asn Gly Thr Arg Ala Ala Leu Ala Gln Ser Glu Arg Ser Ala
                   325                 330                 335
   Val Thr Thr Asp Ala Thr Arg Pro Asp Ala Ala Ser Gln Gly Leu Leu
               340                 345                 350
   Arg Pro Ser Asp Ser His Ser Leu Ser Ser Phe Thr Gln Cys Pro Gln
           355                 360                 365
   Asp Ser Ala Leu Glu Phe Cys Arg Tyr Pro Ala Gln Trp Arg Pro Leu
       370                 375                 380
   Glu Ser Arg Gly Pro Phe Glu Gln Lys Leu Ile Ser Glu Glu Asp Leu
   385                 390                 395                 400
   Asn Met His Thr Gly His His His His His His
   405 410

### MOUSE BACH GENOMIC SEQUENCE (SEQ ID NO: 10)

The genomic sequence of mouse BACH is shown below (SEQ ID NO: 10); this corresponds to the entire genomic contig used to prepare BACH knockout mice. Regions corresponding to the primers are annotated.

Where the term "a mouse BACH genomic sequence" is used in this document, it should be understood as a reference to this sequence.

## Claims

1. An *in vitro* method of identifying a molecule suitable for the treatment or alleviation of pain or a disorder of urogenital function, the method comprising exposing a candidate molecule to a cell expressing a BACH polypeptide comprising an amino acid sequence shown in SEQ ID NO: 3, SEQ ID NO: 5, SEQ ID NO: 7 or SEQ ID NO: 9 or a functionally equivalent sequence having at least 90% sequence identity thereto and determining if a candidate molecule is an agonist or antagonist of the BACH polypeptide.

2. A method according to claim 1, in which a change in intracellular cyclic AMP (cAMP) or calcium levels is detected.

3. A method according to claim 1 or 2, in which a decrease in intracellular cyclic AMP levels is detected to identify an antagonist of BACH polypeptide.

4. A method according to claim 1 or 2, in which an increase in cyclic AMP levels is detected to identify an agonist of BACH polypeptide.

5. A method for providing an indication useful in the diagnosis of or a determination of susceptibility to pain or a disorder of urogenital function in an individual, the method comprising detecting a change in the expression pattern or level of a BACH polypeptide having an amino acid sequence shown in SEQ ID NO: 3, SEQ ID NO: 5, SEQ ID NO: 7 or SEQ ID NO: 9 or a functionally equivalent sequence having at least 90% sequence identity thereto, in a sample from the individual.

6. A method for providing an indication useful in the diagnosis of or a determination of susceptibility to pain or a disorder of urogenital function in an individual, the method comprising detecting a polymorphism in a BACH polynucleotide comprising a nucleic acid sequence shown in SEQ ID NO: 1, SEQ ID NO: 2, SEQ ID NO: 4 or SEQ ID NO: 10 or a functionally equivalent sequence having at least 90% sequence identity thereto, in a sample from the individual.

7. A method according to any one of claims 1 to 4, in which the agonist or antagonist comprises an antibody capable of binding specifically to a BACH polypeptide having an amino acid sequence shown in SEQ ID NO: 3, SEQ ID NO: 5, SEQ ID NO: 7 or SEQ ID NO: 9 or a functionally equivalent sequence having at least 90% sequence identity thereto.

8. Use of a non-human animal which displays a decrease in sensitivity to pain or a hypoactive bladder when compared to a wild-type animal, the non-human animal being a transgenic animal having a functionally disrupted endogenous BACH gene, wherein the BACH gene comprises a nucleic acid sequence shown in SEQ ID NO: 1, SEQ ID NO: 2, SEQ ID NO: 4, SEQ ID NO: 10 or a functionally equivalent sequence having at least 90% sequence identity thereto, to identify an agonist or antagonist of the BACH polypeptide for the treatment or alleviation of pain or a disorder of urogenital function.

9. The use according to claim 8, wherein the non-human animal has a deletion in the BACH gene or a portion thereof.

10. The use according to any one of claims 8 to 9 wherein the non-human animal is a mouse.

11. The use of any one of claim 8 to 10 wherein the non-human animal is a model for pain or a disorder of urogenital function.

## Patentansprüche

1. In vitro-Verfahren der Identifizierung eines für die Behandlung oder Linderung von Schmerzen oder Störungen der Urogenitalfunktion geeigneten Moleküls, umfassend
- Inkontaktbringen eines Kandidatenmoleküls mit einer Zelle, die ein BACH-Polypeptid mit einer in SEQ ID NO: 3, SEQ ID NO: 5, SEQ ID NO: 7.oder SEQ ID NO: 9 gezeigten Aminosäuresequenz oder einer funktionell äquivalenten Aminosäuresequenz, deren Sequenz damit zu mindestens 90% identisch ist, exprimiert, und
- Feststellung, ob ein Kandidatenmolekül ein Agonist oder Antagonist für das BACH-Polypeptid ist.

2. Verfahren nach Anspruch 1, wobei eine Veränderung im intrazellulären zyklischen AMP (cAMP) oder Kalziumgehalt nachgewiesen wird.

3. Verfahren nach Anspruch 1 oder 2, wobei eine Abnahme des intrazellulären zyklischen AMP nachgewiesen wird, um einen Antagonisten des BACH-Polypeptids zu identifizieren.

4. Verfahren nach Anspruch 1 oder 2, wobei eine Zunahme des intrazellulären zyklischen AMP nachgewiesen wird, um einen Agonisten des BACH-Polypeptids zu identifizieren.

5. Verfahren zur Gewinnung eines Hinweises, der bei der Diagnose oder bei der Feststellung einer Anfälligkeit für Schmerzen oder Störungen der Urogenitalfunktion bei einem Individuum brauchbar ist, umfassend den Nachweis einer Veränderung im Expressionsmuster oder im Niveau eines BACH-Polypeptids mit einer in SEQ ID NO: 3, SEQ ID NO: 5, SEQ ID NO: 7 oder SEQ ID NO: 9 gezeigten Aminosäuresequenz oder einer funktionell äquivalenten Aminosäuresequenz, deren Sequenz damit zu mindestens 90% identisch ist, in einer Probe von diesem Individuum.

6. Verfahren zur Gewinnung eines Hinweises, der bei der Diagnose oder bei der Feststellung einer Anfälligkeit für Schmerzen oder Störungen der Urogenitalfunktion bei einem Individuum brauchbar ist, umfassend den Nachweis eines Polymorphismus bei einem BACH-Polynukleotid mit einer in SEQ ID NO: 1, SEQ ID NO: 2, SEQ ID NO: 4 oder SEQ ID NO: 10 gezeigten Nukleinsäuresequenz oder einer funktionell äquivalenten Nukleinsäuresequenz, deren Sequenz damit zu mindestens 90% identisch ist, in einer Probe von diesem Individuum.

7. Verfahren nach einem der Ansprüche 1 bis 4, wobei der Agonist oder Antagonist einen Antikörper umfasst, der spezifisch an ein BACH-Polypeptid mit einer in SEQ ID NO: 3, SEQ ID NO: 5, SEQ ID NO: 7.oder SEQ ID NO: 9 gezeigten Aminosäuresequenz oder einer funktionell äquivalenten Aminosäuresequenz, deren Sequenz damit zu mindestens 90% identisch ist, binden kann.

8. Verwendung eines nichtmenschlichen Tiers, welches im Vergleich zum Wildtyp dieses Tiers eine Verminderung der Empfindlichkeit für Schmerzen oder hyperaktive Blase zeigt, wobei das nichtmenschliche Tier ein transgenes Tier mit einem funktionell zerstörten endogenen BACH-Gen ist, wobei das BACH-Gen eine in SEQ ID NO: 1, SEQ ID NO: 2, SEQ ID NO: 4 oder SEQ ID NO: 10 gezeigten Nukleinsäuresequenz oder einer funktionell äquivalenten Nukleinsäuresequenz, deren Sequenz damit zu mindestens 90% identisch ist, umfaßt, zur Identifikation eines Agonisten oder Antagonisten des BACH-Polypeptids für die Behandlung oder Linderung von Schmerzen oder Störungen der Urogenitalfunktion.

9. Verwendung nach Anspruch 8, wobei das nichtmenschliche Tier eine Deletion im BACH-Gen oder einem seiner Abschnitte aufweist.

10. Verwendung nach Anspruch 8 oder 9, wobei das nichtmenschliche Tier eine Maus ist.

11. Verwendung nach einem der Ansprüche 8 bis 10, wobei das nichtmenschliche Tier ein Modell für Schmerzen oder Störungen der Urogenitalfunktion ist.

## Revendications

1. Procédé *in vitro* pour identifier une molécule appropriée pour le traitement ou le soulagement de la douleur ou d'un trouble de la fonction uro-génitale, le procédé consistant à exposer une molécule candidate à une cellule exprimant un polypeptide BACH comprenant une séquence d'acides aminés représentée dans SEQ ID NO: 3, SEQ ID NO: 5, SEQ ID NO: 7 ou SEQ ID NO: 9, ou une séquence fonctionnellement équivalente ayant au moins 90 % d'identité de séquence avec celles-ci, et à déterminer si une molécule candidate est un agoniste ou un antagoniste du polypeptide BACH.

2. Procédé selon la revendication 1, dans lequel une modification des taux d'AMP cyclique (AMPc) intracellulaire ou de calcium est détectée.

3. Procédé selon la revendication 1 ou 2, dans lequel une réduction des taux d'AMP cyclique intracellulaire est détectée afin d'identifier un antagoniste d'un polypeptide BACH.

4. Procédé selon la revendication 1 ou 2, dans lequel une augmentation des taux d'AMP cyclique est détectée afin d'identifier un agoniste d'un polypeptide BACH.

5. Procédé pour obtenir une indication utile dans le diagnostic ou dans une détermination d'une sensibilité à la douleur ou un trouble de la fonction uro-génitale chez un individu, le procédé consistant à détecter une modification du motif ou du taux d'expression d'un polypeptide BACH ayant une séquence d'acides aminés représentée dans SEQ ID NO: 3, SEQ ID NO: 5, SEQ ID NO: 7 ou SEQ ID NO: 9, ou une séquence fonctionnellement équivalente ayant au moins 90 % d'identité de séquence avec celles-ci, dans un échantillon issu de l'individu.

6. Procédé pour obtenir une indication utile dans le diagnostic ou dans une détermination d'une sensibilité à la douleur ou un trouble de la fonction uro-génitale chez un individu, le procédé consistant à détecter un polymorphisme dans un polynucléotide BACH comprenant une séquence d'acide nucléique représentée dans SEQ ID NO: 1, SEQ ID NO: 2, SEQ ID NO: 4 ou SEQ ID NO: 10, ou une séquence fonctionnellement équivalente ayant au moins 90 % d'identité de séquence avec celles-ci, dans un échantillon issu de l'individu.

7. Procédé selon l'une quelconque des revendications 1 à 4, dans lequel l'agoniste ou l'antagoniste comprend un anticorps capable de se lier spécifiquement à un polypeptide BACH ayant une séquence d'acides aminés représentée dans SEQ ID NO: 3, SEQ ID NO: 5, SEQ ID NO: 7 ou SEQ ID NO: 9, ou une séquence fonctionnellement équivalente ayant au moins 90 % d'identité de séquence avec celles-ci.

8. Utilisation d'un animal non humain qui présente une réduction de sensibilité à la douleur ou une vessie hypoactive par comparaison à un animal de type sauvage, l'animal non humain étant un animal transgénique ayant un gène BACH endogène fonctionnellement perturbé, où le gène BACH comprend une séquence d'acide nucléique représentée dans SEQ ID NO:1, SEQ ID NO:2, SEQ ID NO:4 ou SEQ ID NO: 10, ou une séquence fonctionnellement équivalente ayant au moins 90 % d'identité de séquence avec celles-ci, afin d'identifier un agoniste ou un antagoniste du polypeptide BACH pour le traitement ou le soulagement de la douleur ou d'un trouble de la fonction uro-génitale.

9. Utilisation selon la revendication 8, dans laquelle l'animal non humain comporte une délétion dans le gène BACH ou une partie de celui-ci.

10. Utilisation selon l'une quelconque des revendications 8 à 9, dans laquelle l'animal non humain est une souris.

11. Utilisation selon l'une quelconque des revendications 8 à 10, dans laquelle l'animal non humain est un modèle pour la douleur ou un trouble de la fonction uro-génitale.
